# EUROPEAN PATENT APPLICATION

(11) **EP 4 295 903 A2**
(43) Date of publication of application: **27.12.2023**
(21) Application number: 23184825.0
(22) Date of filing: 12.01.2018
(51) Int. Cl.: A61P 3/00

(54) **COMPOSITIONS AND METHODS FOR TREATING FARBER DISEASE**

(30) Priority: 13.01.2017 US 201762446166 P
(62) Divisional of application: 18739237.8
(71) Applicant: Icahn School of Medicine at Mount Sinai, New York, NY 10029 (US)
(72) Inventor: The designation of the inventor has not yet been filed
(74) Representative: Adamson Jones

(57) **Abstract**

Proteins, compositions, and methods for treating Farber disease are provided.

## Description

### Cross-reference to Related Applications

This application claims priority benefit of U.S. Provisional Patent Application Serial No. 62/446,166, filed January 13, 2017, which is hereby incorporated by reference in its entirety.

### Background

Farber disease, a lysosomal storage disorder (LSD), is a condition that was first described in 1952 in a 14-month-old infant with granulomatous lesions on multiple joints and evidence of lipid storage. Over the ensuing decade other similar cases were described, all of whom had similar lesions and often exhibited a characteristic "hoarse" cry or voice due to the presence of lesions on the larynx. The involvement of other organ systems in some of these patients, including the lung, liver, spleen and central nervous system (CNS), also was noted.

Previously, treatment for Farber disease patients has been symptomatic and principally aimed at reducing pain. Hematopoietic stem cell transplantation (HSCT) has been undertaken in several patients, and overall the outcome has been positive provided that the transplant procedure itself was successful. Such transplanted patients exhibit significant reduction in pain, increased motility and mobility, and in some cases shrinking and complete resolution of the subcutaneous nodules. However, successful transplantation requires histocompatible donor cells, and exposes patients to invasive and potentially dangerous immunosuppressant regimes. One alternative to HSCT is gene therapy in which autologous donor cells are transduced with a vector expressing the therapeutic protein, obviating the need for histocompatible donors. This approach has been evaluated in the Farber disease knock-in mice and resulted in reduction of tissue ceramides and macrophage infiltration. However, there continues to be a need for improved therapies for treating Farber disease. The present subject matter fulfills other needs as well as will be discussed herein.

### Summary

Embodiments disclosed herein provide methods of treating Farber disease and/or related conditions thereof in a subject in need thereof, the methods comprising administering to the subject a pharmaceutical composition comprising an effective amount of a recombinant human acid ceramidase. In some aspects, the recombinant human acid ceramidase that is administered has no detectable sphingomyelinase activity.

Embodiments disclosed herein provide methods of treating Farber disease in a subject in need thereof, the methods comprising administering to the subject a pharmaceutical composition comprising a recombinant human acid ceramidase in an effective amount of about 0.1 mg/kg to about 50 mg/kg.

Embodiments disclosed herein provide methods of reducing lipogranulomas in a subject with, or suspected of having, Farber disease, the methods comprising administering to the subject a pharmaceutical composition comprising a recombinant human acid ceramidase in an effective amount of about 0.1 mg/kg to about 50 mg/kg.

Embodiments disclosed herein provide methods of reducing spleen weight in a subject with, or suspected of having, Farber disease, the methods comprising administering to the subject a pharmaceutical composition comprising a recombinant human acid ceramidase in an effective amount of about 0.1 mg/kg to about 50 mg/kg.

Embodiments disclosed herein provide methods of reducing ceramide in a subject with, or suspected of having, Farber disease, the methods comprising administering to the subject a pharmaceutical composition comprising a recombinant human acid ceramidase in an effective amount of about 0.1 mg/kg to about 50 mg/kg

Embodiments disclosed herein provide methods, of increasing sphingosine in a subject with, or suspected of having, Farber disease, the method comprising administering to the subject a pharmaceutical composition comprising a recombinant human acid ceramidase in an effective amount of about 0.1 mg/kg to about 50 mg/kg.

Embodiments disclosed herein provide methods of treating Farber disease in a subject in need thereof, the methods comprising expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg.

Embodiments disclosed herein provide methods of reducing lipogranulomas in a subject with, or suspected of having, Farber disease, the methods comprising: expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg.

Embodiments disclosed herein provide methods of reducing spleen weight in a subject with, or suspected of having, Farber disease, the methods comprising expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg.

Embodiments disclosed herein provide methods of reducing ceramide in a subject with, or suspected of having, Farber disease, the methods comprising expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg.

Embodiments disclosed herein provide methods of increasing sphingosine in a subject with, or suspected of having, Farber disease, the methods comprising expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg.

Embodiments disclosed herein provide methods of treating Farber disease in a subject in need thereof, the methods comprising administering to the subject a pharmaceutical composition comprising a recombinant human acid ceramidase (rhAC) in an effective amount of about 3, 10, or 50 mg/kg in, for example, once a week, once every two weeks, or once a month repeat dosages for the duration of subject's life.

Embodiments disclosed herein provide methods of treating Farber disease in a subject in need thereof, the methods comprising administering to the subject a pharmaceutical composition comprising a recombinant human acid ceramidase (rhAC) in an effective amount of about 1 mg to about 5 mg/kg or about 2 mg/kg to about 5 mg/kg in, for example, once a week, once every two weeks, or once a month repeat dosages for at least 10 or at least 20 weeks, for 28 weeks, or for the duration of subject's life. In some embodiments, the administration is by intravenous infusion. In one embodiment, the method of treating Farber disease in a subject in need thereof comprises administering to the subject a pharmaceutical composition comprising a recombinant human acid ceramidase (rhAC) in an effective amount of about 1 mg to about 5 mg/kg or about 2 mg/kg to about 5 mg/kg in, for example, once a week, once every two weeks, or once a month repeat dosages for at least 10 or 20 weeks, for 28 weeks, or for the duration of subject's life.

In certain aspects of the invention, the treatment is started when subject is under one year of age.

In aspects of the invention, the treatment is started when the subject is between 1 and 5 years of age.

### Brief Description

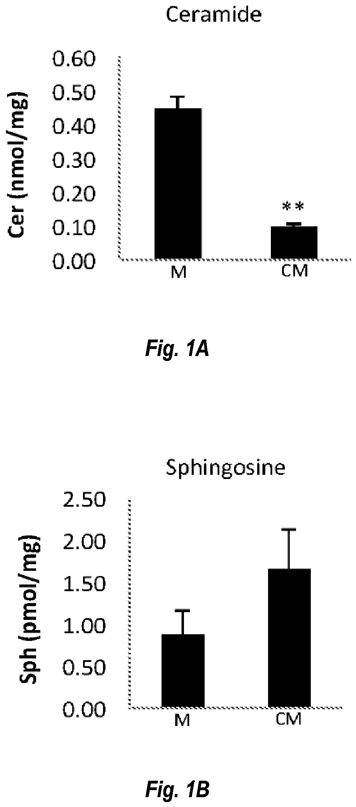

**Figs. 1A and 1B****.** Effect of rhAC on ceramide and sphingosine in Farber disease cells. Serum free conditioned media (CM) was obtained from a Chinese hamster ovarian (CHO) cell line overexpressing rhAC and added to an SV40-transformed skin fibroblast line derived from a Farber disease patient (see Example 1, Materials and Methods). Serum free parental CHO media (M) lacking rhAC was used as a control. After media exchange, the Farber cells were grown for 24 h, harvested, washed 3X with PBS (phosphate-buffered saline), and the amounts of total ceramide (Cer) and sphingosine (Sph) were quantified and expressed per milligram (mg) total cell protein as shown in Figs. 1A and 1B, respectively. **indicates p value <0.01 comparing cells grown with CM to cells grown in M.

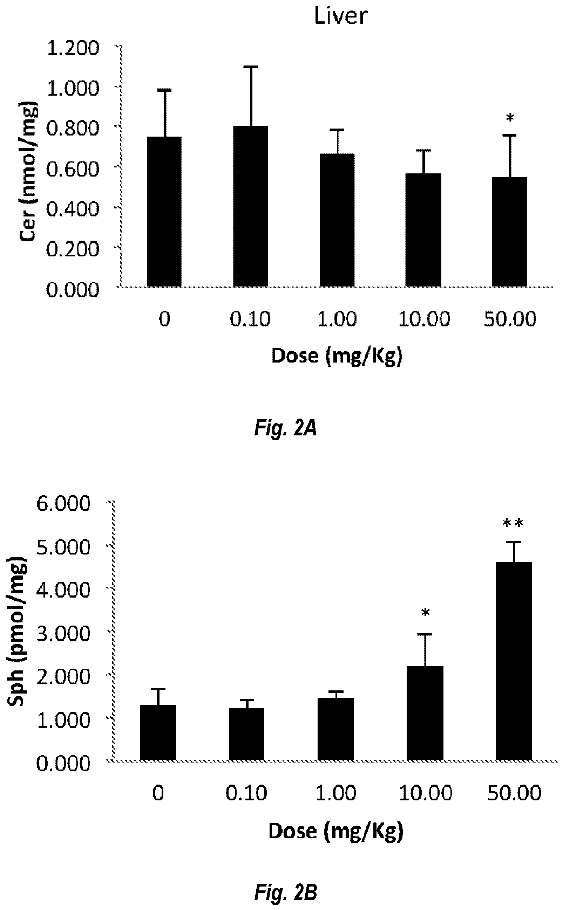

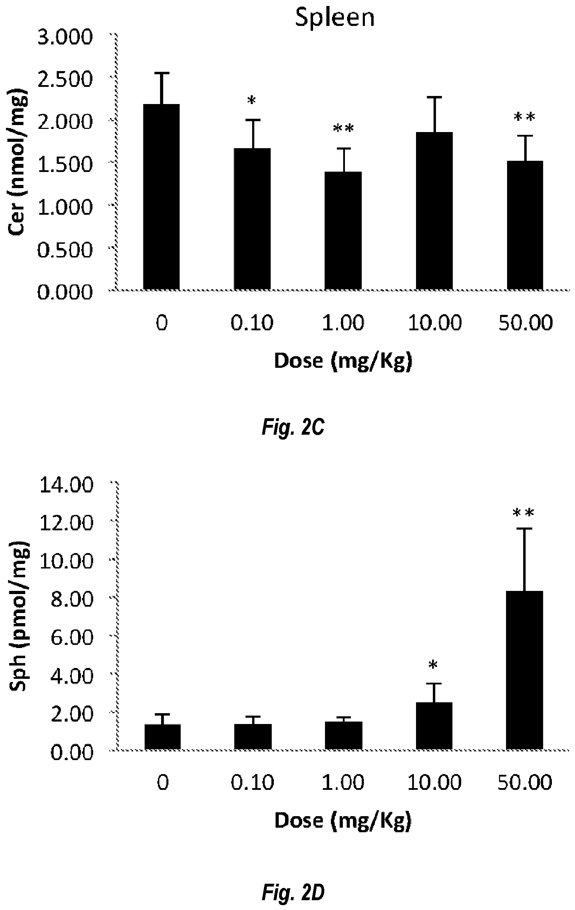

**Figs. 2A-2D****.** Ceramide and sphingosine in the livers and spleens of Farber disease mice after single injection of rhAC. Groups of ~9-week-old Farber disease mice received single i.p. injections of purified rhAC at the indicated doses (n=3/dose). After 24 h the mice were euthanized and the amounts of total ceramide (Cer) and sphingosine (Sph) were quantified and expressed per milligram total protein in tissue extracts from the liver in Figs. 2A (Cer) and 2B (Sph), and the spleen in Figs. 2C (Cer) and 2D (Sph). "0" indicates age-matched Farber mice that were injected with PBS. *indicates p value <0.05 comparing mice injected with rhAC to mice injected with PBS; **indicates p value <0.01.

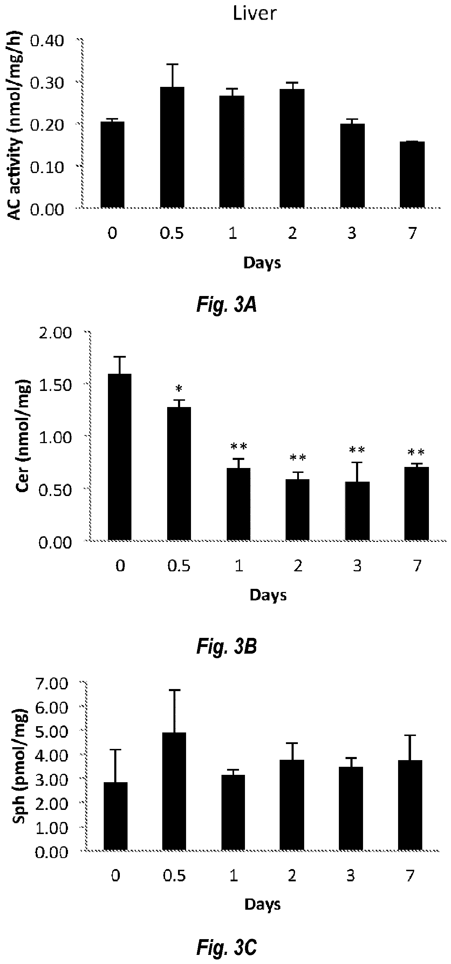

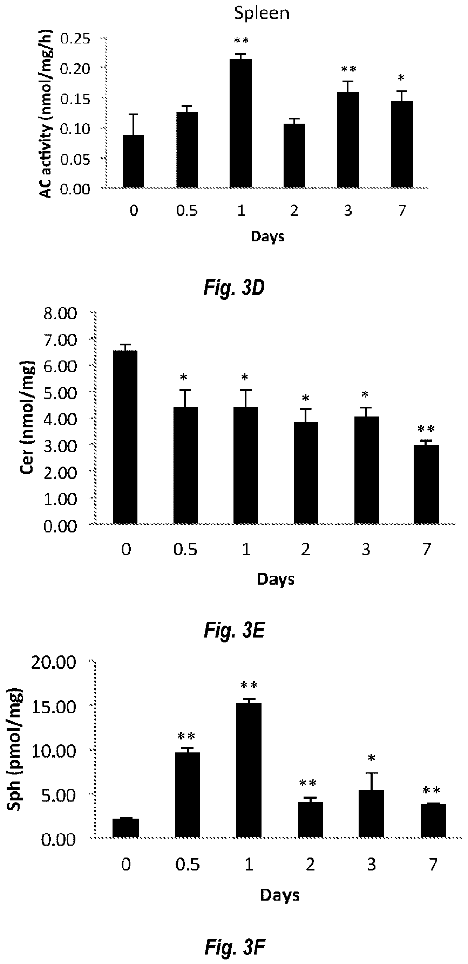

**Figs. 3A-3F****.** Time course of AC activity, ceramide, and sphingosine in the livers (Figs. 3A-3C, respectively) and spleens (Figs. 3D-3F, respectively) of Farber disease mice after single injection of rhAC. Approximately 9-week-old Farber disease mice received single i.p. injections of purified rhAC at a dose of 10 mg/kg. At the indicated times post-injection, mice were euthanized (n=3 per time point), and the amounts of total ceramide (Cer), sphingosine (Sph), and AC activity were quantified and expressed per milligram protein in tissue extracts. FD indicates untreated, age-matched Farber disease mice injected with PBS. *indicates p value <0.05 comparing mice injected with rhAC to mice injected with PBS. **indicates p value <0.01.

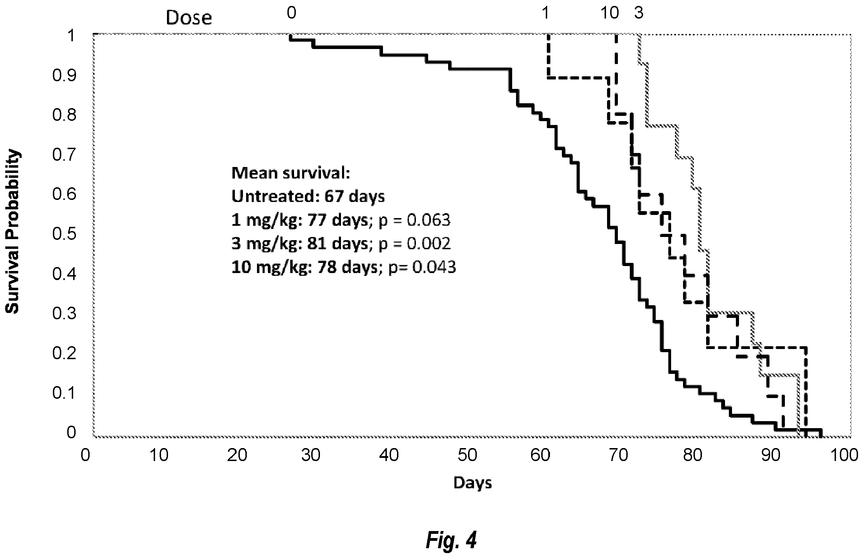

**Fig. 4****.** Survival of Farber disease mice receiving repeat injections of rhAC. Approximately 3-week-old Farber disease mice received once weekly i.p. injections of purified rhAC at doses of 1, 3 and 10 mg/kg (n=9-56/dose). Mice were euthanized when they lost >10% body weight within a one week period. Kaplan-Meir plots were used to indicate survival probability. "0" indicates Farber disease mice receiving once weekly injections of PBS.

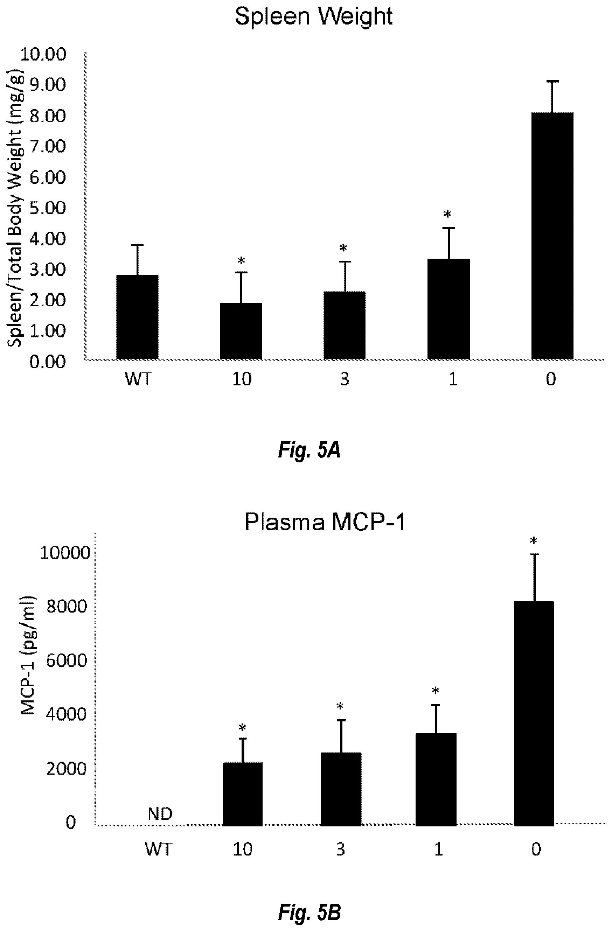

**Figs. 5A-5B****.** Spleen weight and plasma MCP-1 (monocyte chemoattractant protein-1) levels in Farber disease mice receiving repeat injections of rhAC. Approximately 3-week-old Farber disease mice received once weekly i.p. injections of purified rhAC at doses of 1, 3 and 10 mg/kg (n=4-9/dose). Mice were euthanized when they lost >10% body weight within a one week period. Spleen weight **(****Fig. 5A****)** was expressed per total body weight. MCP-1 **(****Fig. 5B****)** was measured in plasma using ELISA kits (see Materials and Methods of Example 1). "0" indicates Farber mice receiving once weekly injections of PBS. The spleen weight and MCP-1 levels in age-matched wild-type mice (WT) also is shown. *indicates p value <0.05 comparing mice treated with rhAC to mice treated with PBS.

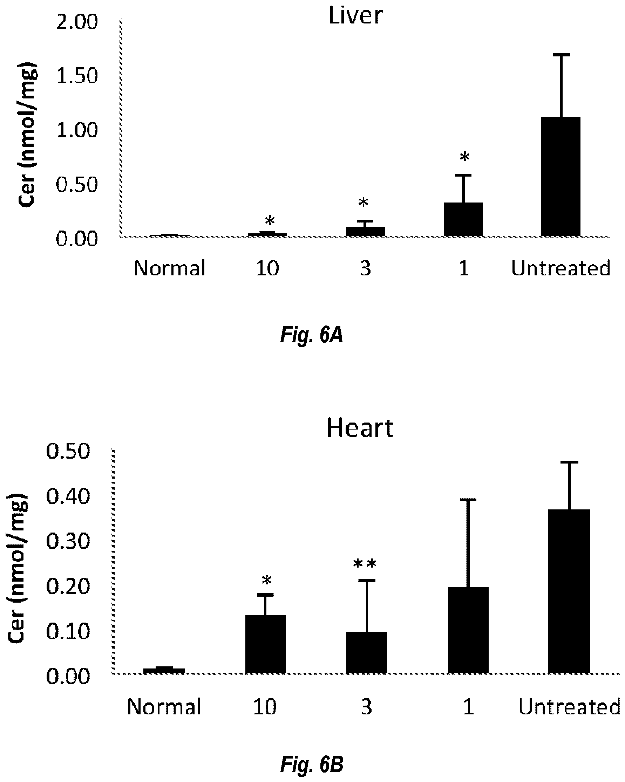

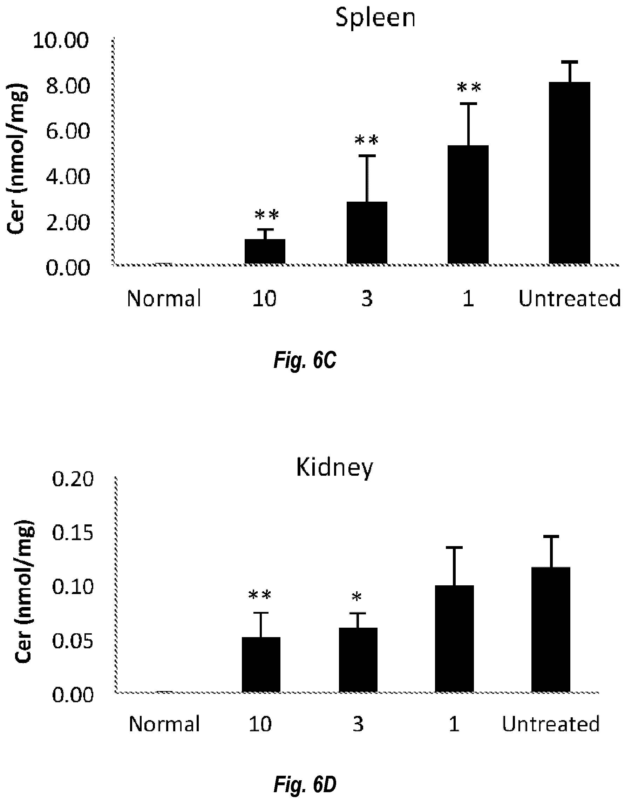

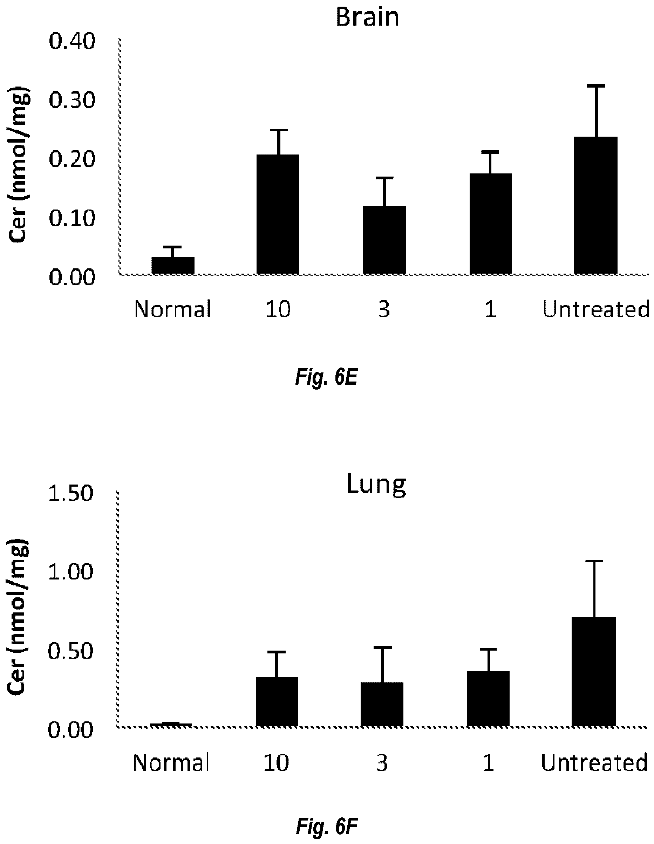

**Figs. 6A-6F****.** Ceramide in tissues of Farber disease mice receiving repeat injections of rhAC. Approximately 3-week-old Farber disease mice received once weekly i.p. injections of purified rhAC at doses of 1, 3 and 10 mg/kg (n=4-10/dose). Mice were euthanized when they lost >10% body weight within a one week period. Total ceramide (Cer) levels were determined in tissue extracts (Fig. 6A liver, Fig. 6B heart, Fig. 6C spleen, Fig. 6D kidney, Fig. 6E brain, and Fig. 6F lung), as described in the Materials and Methods of Example 1. "0" indicates Farber disease mice receiving once weekly injections of PBS. WT indicates age-matched wild-type mice. *indicates p value <0.05 comparing mice treated with rhAC to mice treated with PBS. **indicates p value <0.01.

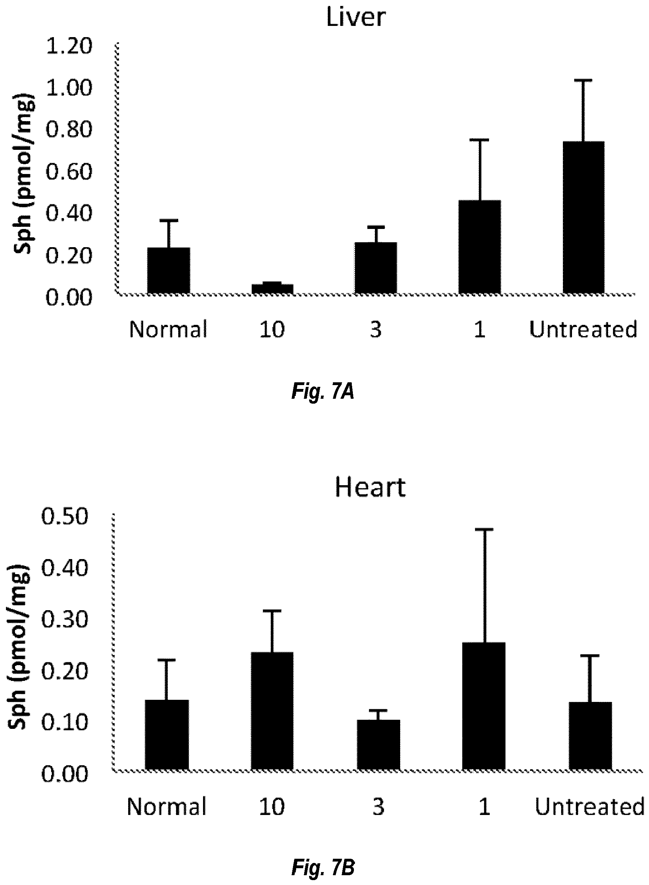

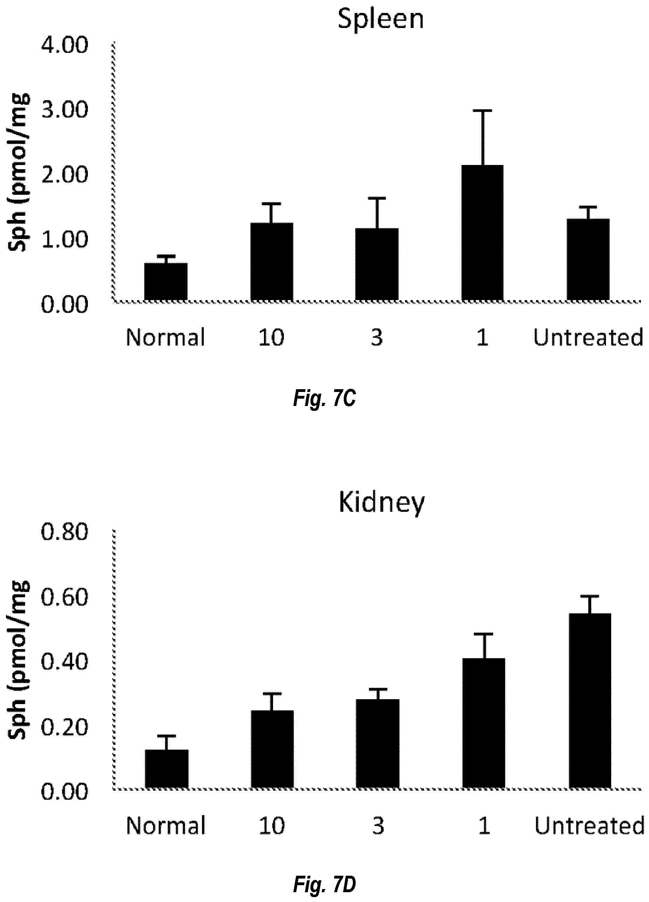

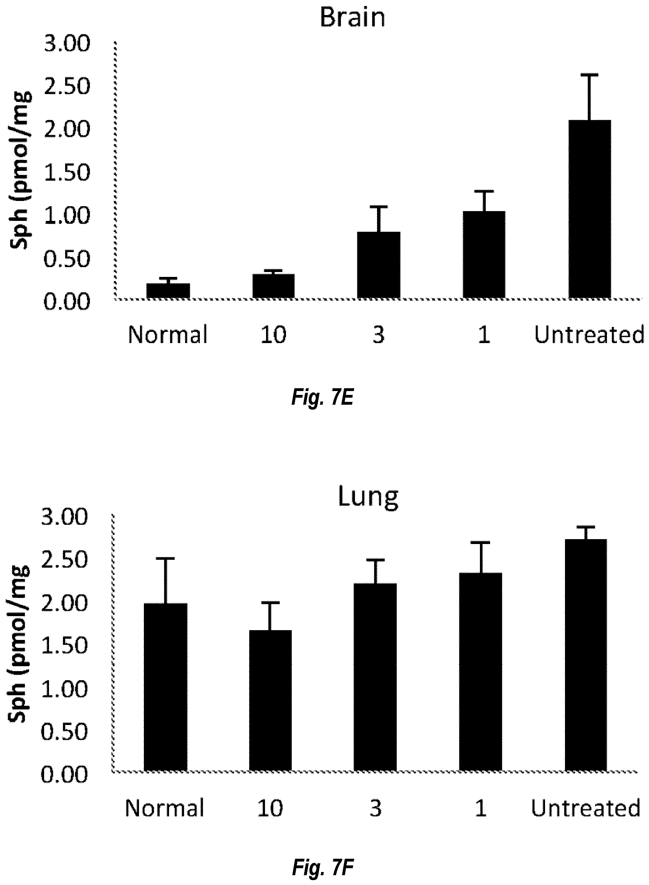

**Figs. 7A-7F****.** Sphingosine in tissues of Farber disease mice receiving repeat injections of rhAC. Approximately 3-week-old Farber disease mice received once weekly i.p. injections of purified rhAC at doses of 1, 3 and 10 mg/kg (n=4-10/dose). Mice were euthanized when they lost >10% body weight within a one week period. Total sphingosine (Sph) levels were determined in tissue extracts (Fig. 7A liver, Fig. 7B heart, Fig. 7C spleen, Fig. 7D kidney, Fig. 7E brain, and Fig. 7F lung), as described in the Materials and Methods of Example 1. "0" indicates Farber disease mice receiving once weekly injections of PBS. WT indicates age-matched wild-type mice. *indicates p value <0.05 comparing mice treated with rhAC to mice treated with PBS. **indicates p value <0.01.

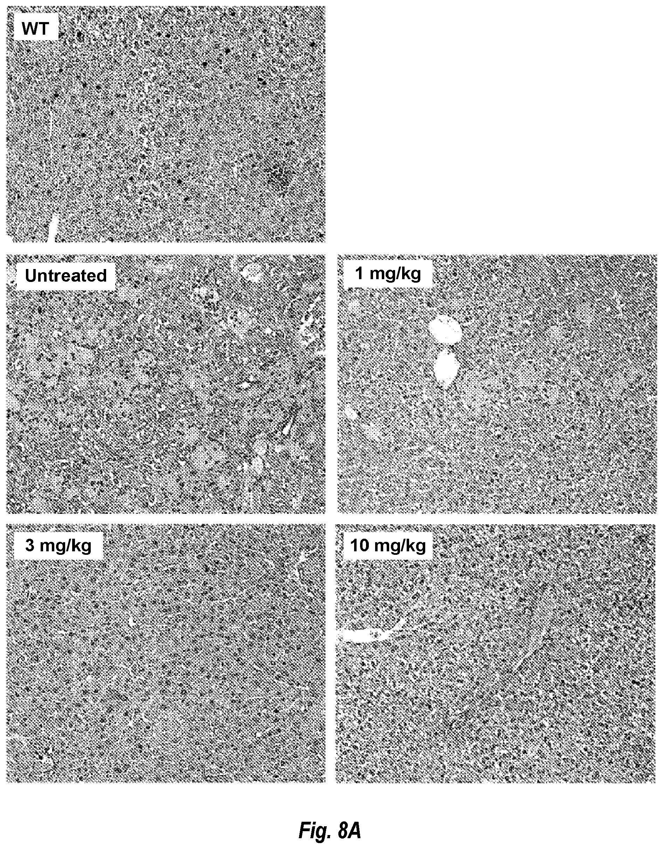

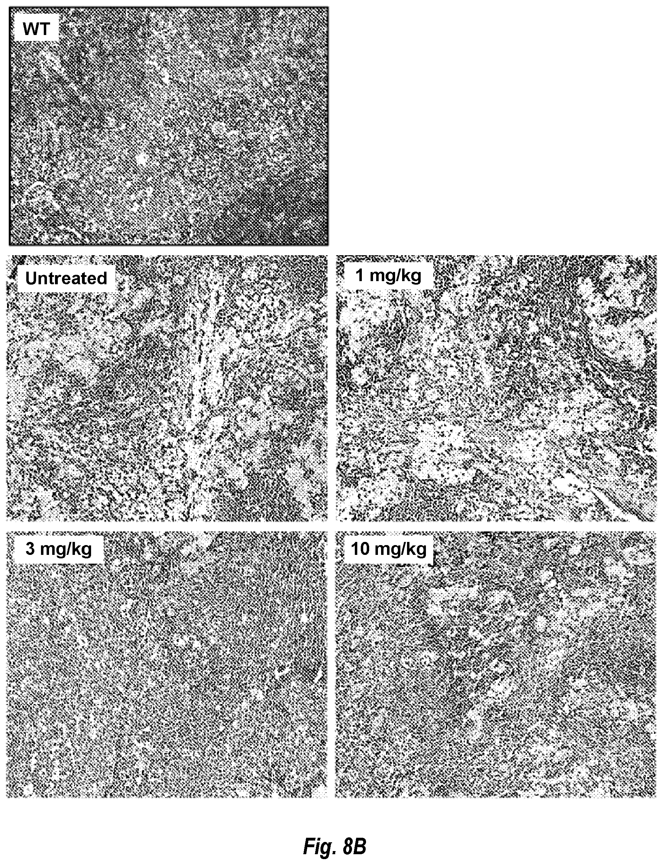

**Fig. 8A** **and** **Fig. 8B****.** Histology in liver and spleen sections from Farber disease mice receiving repeat injections of rhAC. Approximately 3-week-old Farber disease mice received once weekly i.p. injections of purified rhAC at doses of 1, 3 and 10 mg/kg (n=xx/dose). Mice were euthanized when they lost >10% body weight within a one week period. Livers **(****Fig. 8A****)** and spleens **(****Fig. 8B****)** were fixed in formalin, sectioned and subjected to H&E staining. "0" indicates Farber disease mice receiving once weekly injections of PBS. WT indicates age-matched wild-type mice. Representative sections are shown from mice in each dose group. Magnification = 20X.

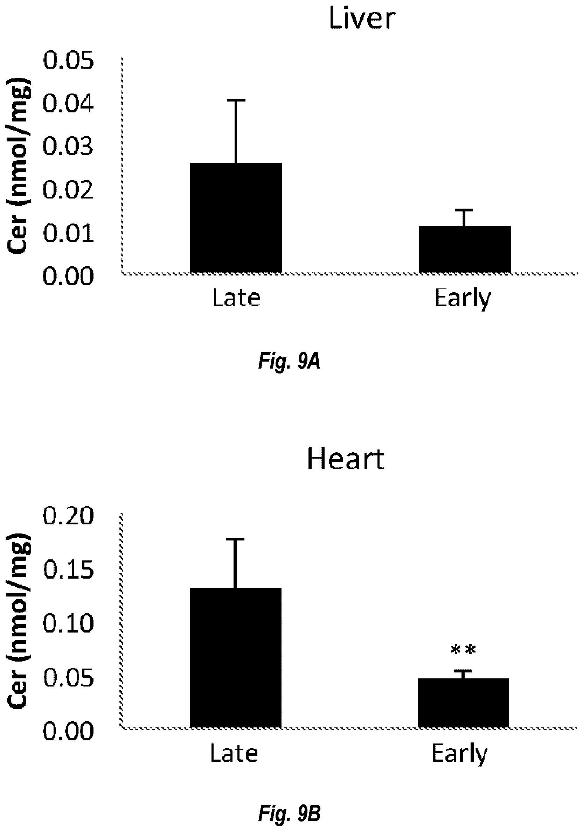

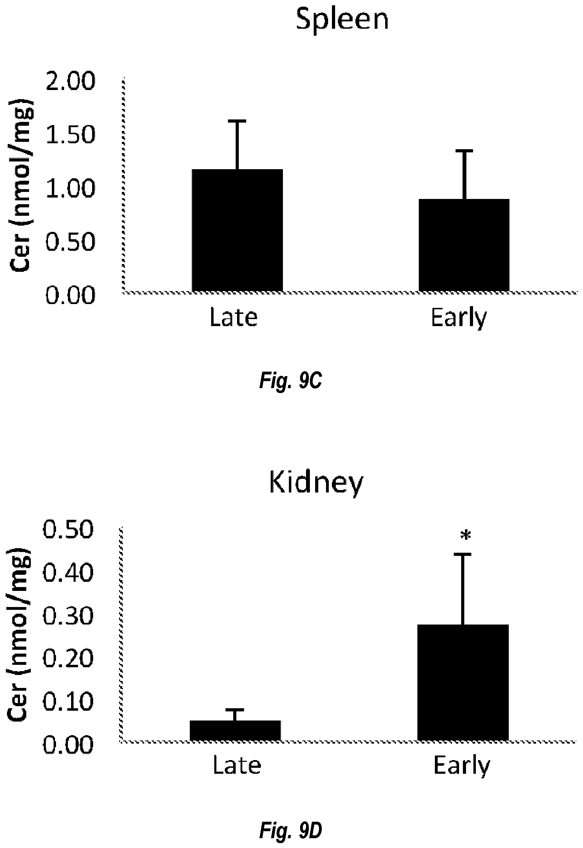

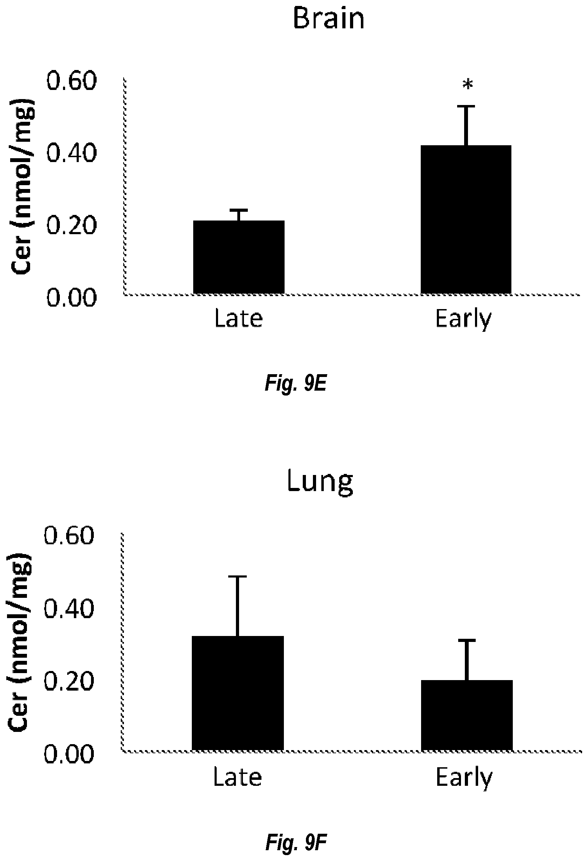

**Figs. 9A-9F****.** Ceramide in tissues of Farber disease mice receiving repeat injections of rhAC starting at two different ages. Farber disease mice received once weekly i.p. injections of purified rhAC (10 mg/kg) starting at 3 days (early) or 3 weeks (late) of age (n=7-11/age). Mice were euthanized when they lost >10% body weight within a one week period. Total ceramide (Cer) levels were determined in tissue extracts (Fig. 9A liver, Fig. 9B heart, Fig. 9C spleen, Fig. 9D kidney, Fig. 9E brain, and Fig. 9F lung), as described in the Materials and Methods of Example 1. *indicates p value <0.05 comparing mice treated early to late. **indicates p value <0.01.

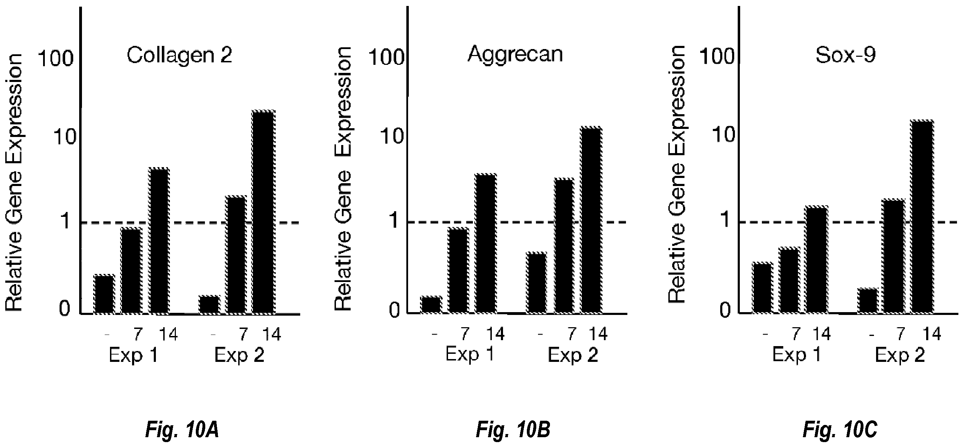

**Figs. 10A-10C****.** Effect of rhAC on chondrocytes from Farber disease mice. Primary chondrocytes were obtained from the humeri of 4-6-week old Farber disease mice. Cells from 5-10 mice were pooled to establish cultures, and grown in standard media (-) or media containing purified rhAC (12.5 ug/ml) for 7 or 14 days. rhAC was added once at the time of cell plating. qPCR was then used to assess the expression of three chondrogenic marker genes, collagen 2 (Fig. 10A), aggrecan (Fig. 10B), and Sox-9 (Fig. 10C). The dotted lines indicate the expression of these genes in chondrocytes grown from healthy mice in standard media. The results of two separate experiments (Experiments 1 and 2) are shown.

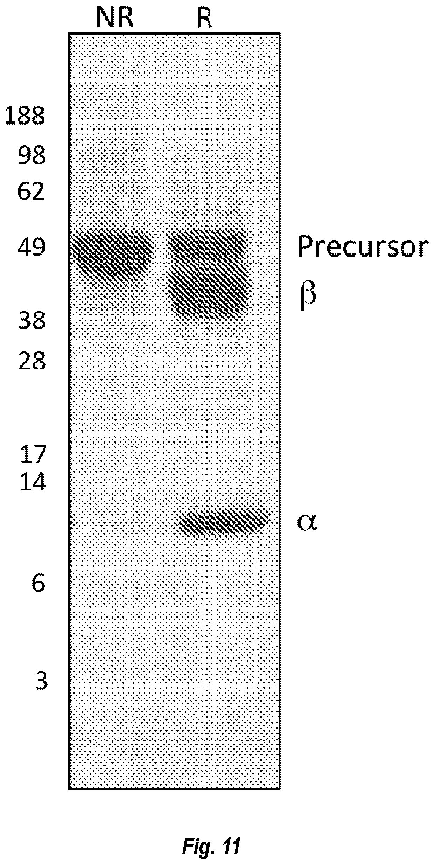

**Fig. 11****.** Representative SDS PAGE analysis of rhAC. rhAC was purified from the media of an overexpressing CHO cell line and subjected to SDS PAGE under non-reducing (NR) and reducing (R) conditions. Staining was with SimpleBlue^{®}. Molecular weights (in kDa) according to the migration of commercially supplied molecular weight standards are shown to the left. Bands corresponding to rhAC precursor, alpha and beta subunits also are indicated.

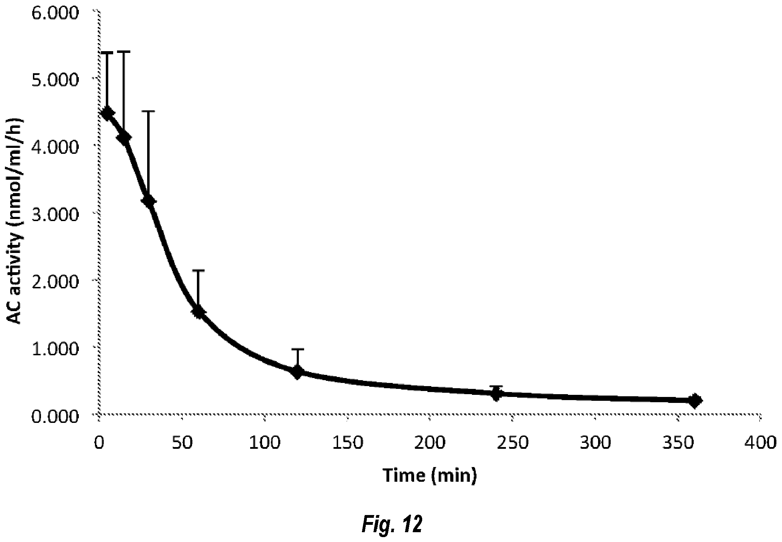

**Fig. 12****.** Plasma clearance of rhAC activity after tail vein injection. rhAC (10 mg/kg) was injected into the tail vein of 4-6-week old Farber disease mice. At various times, post-injection mice were euthanized (n=3/time point) and AC activity was determined in the plasma.

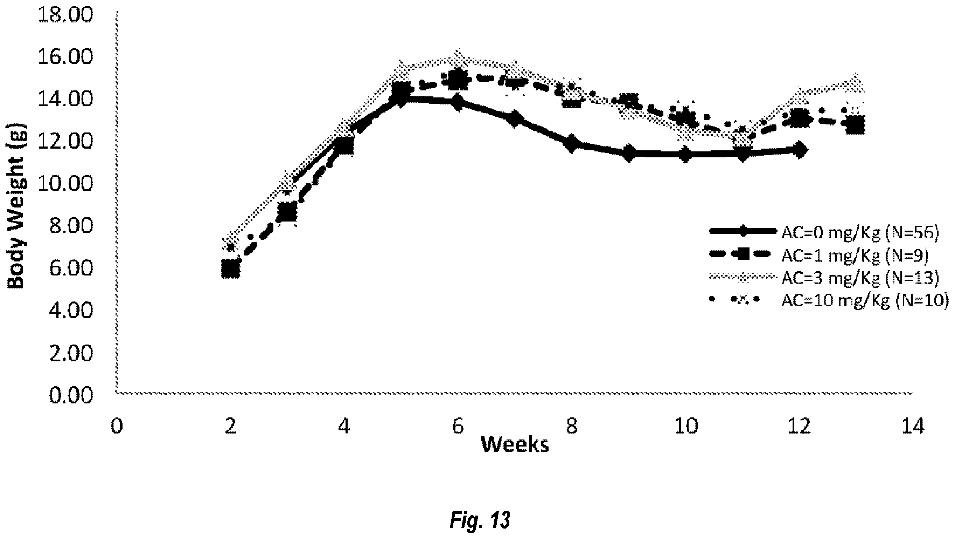

**Fig. 13****.** Weight of Farber disease mice receiving repeat injections of rhAC. Approximately 3-week-old Farber disease mice received once weekly i.p. injections of purified rhAC (AC) at doses of 1, 3 and 10 mg/kg (n=9-56/dose). Mice were euthanized when they lost >10% body weight within a one week period. "0" indicates Farber disease mice receiving once weekly injections of PBS. The average weight of surviving mice at the indicated ages is graphed.

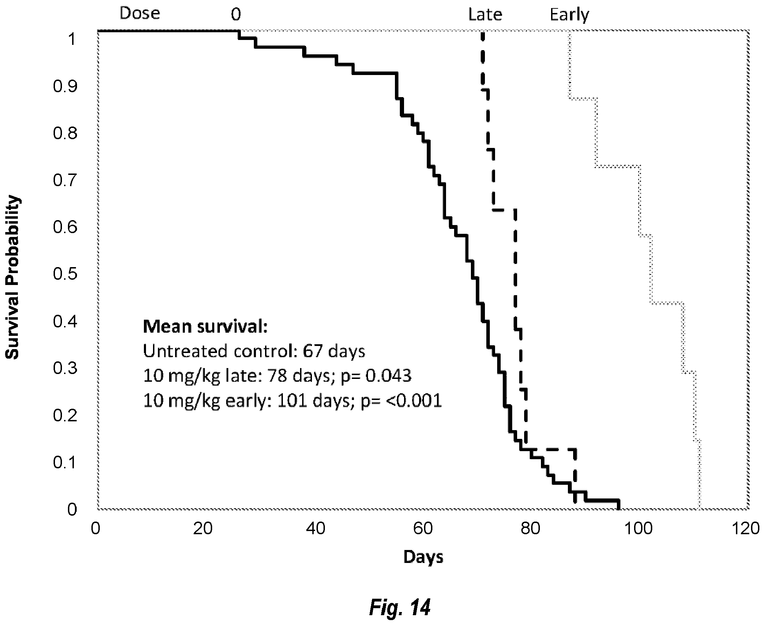

**Fig. 14****.** Survival of Farber disease mice receiving repeat injections of rhAC starting at two different ages. Farber disease mice received once weekly i.p. injections of purified rhAC (10 mg/kg) starting at 3 days (early) or 3 weeks (late) of age (n=7-10/age). Mice were euthanized when they lost >10% body weight within a one week period. Kaplan-Meir plots were used to indicate survival probability. "0" indicates Farber disease mice receiving once weekly injection of PBS.

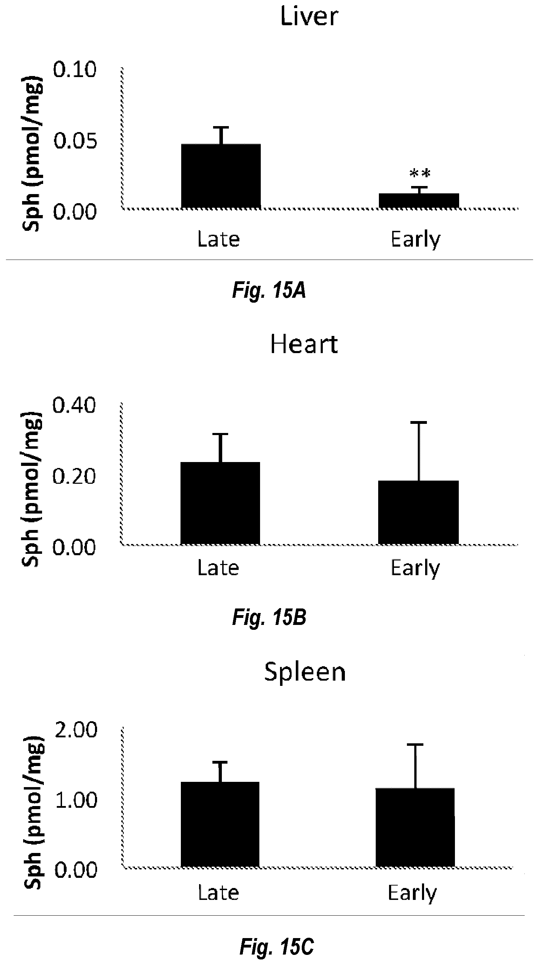

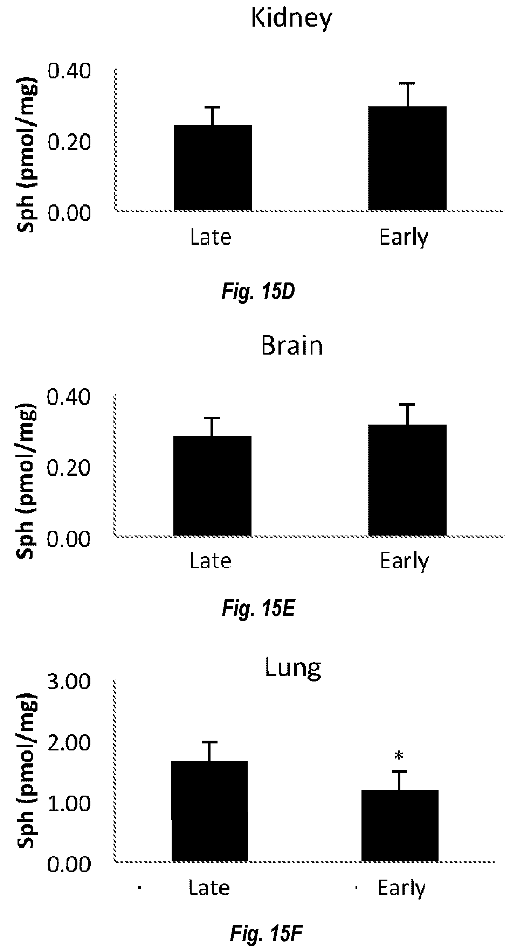

**Figs. 15A-15F****.** Sphingosine in tissues of Farber disease mice receiving repeat injections of rhAC starting at two different ages. Farber disease mice received once weekly i.p. injections of purified rhAC (10 mg/kg) starting at 3 days (early) or 3 weeks (late) of age (n=7-11/age). Mice were euthanized when they lost >10% body weight within a one week period. Sphingosine (Sph) levels were determined in tissue extracts (Fig. 15A liver, Fig. 15B heart, Fig. 15C spleen, Fig. 15D kidney, Fig. 15E brain, and Fig. 15F lung), as described in the Materials and Methods of Example 1. *indicates p value <0.05 comparing mice treated early to late. **indicates p value <0.01.

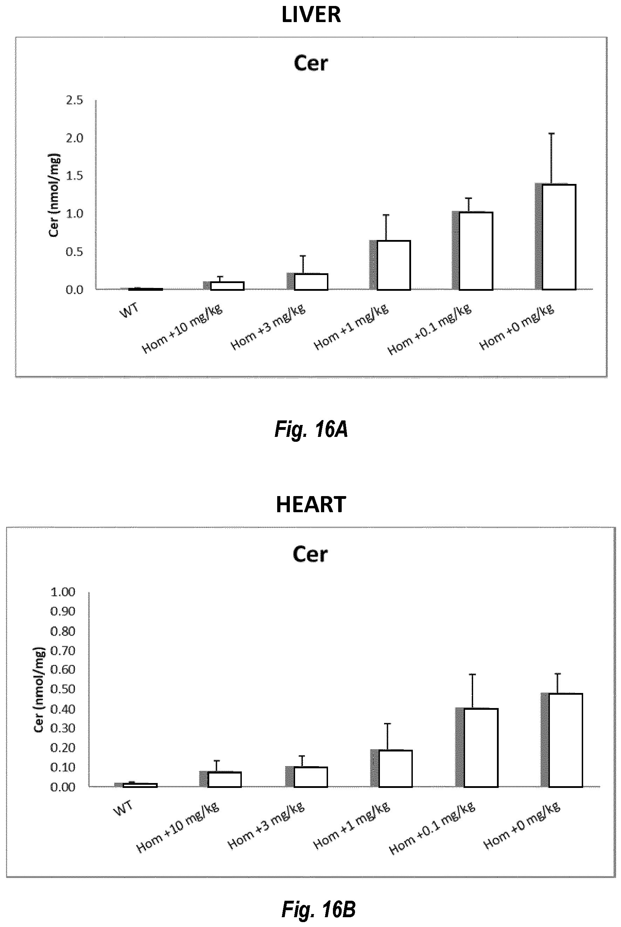

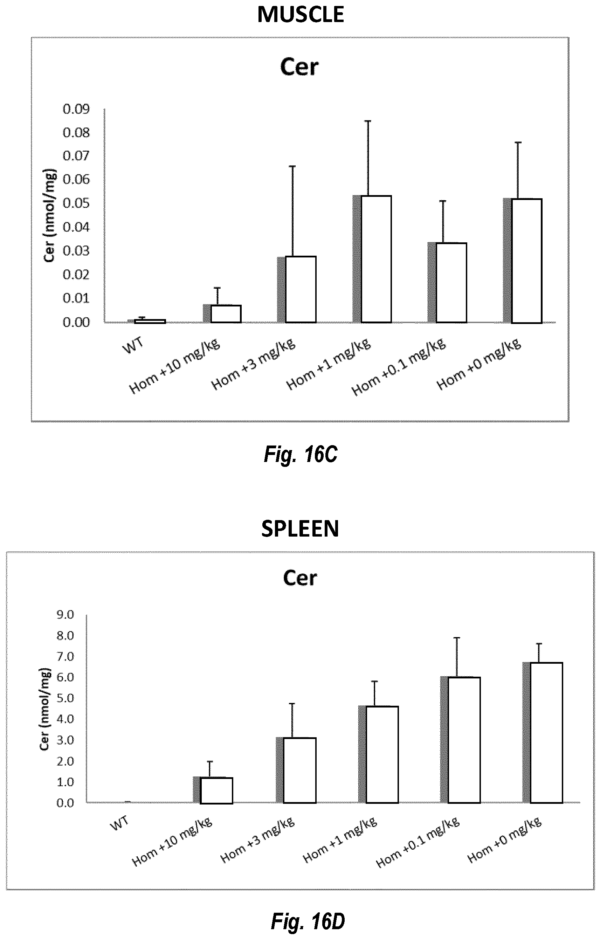

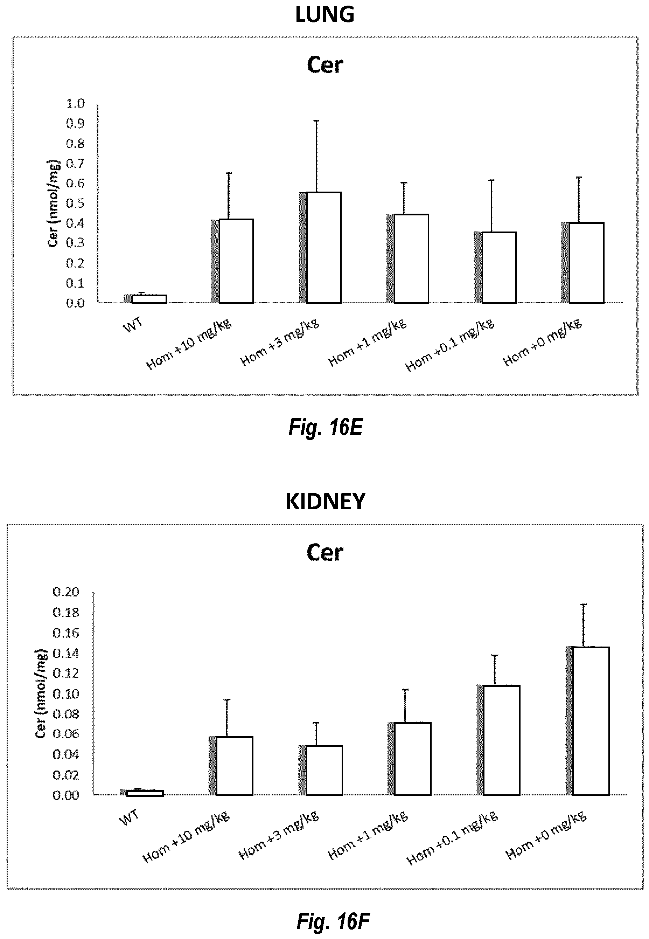

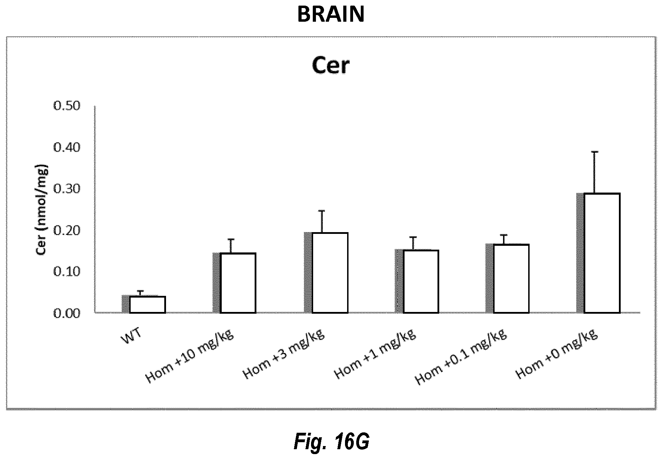

**Figs. 16A-16G****.** Ceramide in tissues of wild-type (WT) mice and Farber disease mice (horn) (homozygous for the asah1^{P361R/P361R} mutation) receiving once weekly injections of purified rhAC at different dosages of 0, 0.1, 1, 3, and 10 mg/kg/dose for 6 weeks starting at 3-4 weeks of age. Mice were euthanized 48 hours after the 6th dose. Ceramide (Cer) levels were determined in tissue extracts (Fig. 16A liver, Fig. 16B heart, Fig. 16C muscle, Fig. 16D spleen, Fig. 16E lung, 16F kidney, and Fig. 16G brain), as described in the Materials and Methods of Example 2.

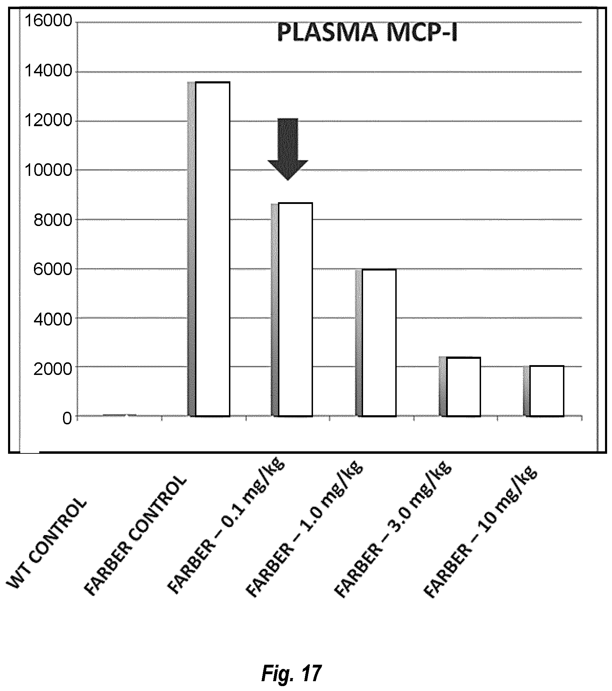

**Fig. 17****.** Plasma monocyte chemoattractant protein (MCP)-1, a systemic marker of inflammation, in terminal blood collected 48 hours after the final dose in treated and control Farber disease mice (Farber), as compared to wild-type (WT) control. A clear dose response is shown as a transition away from overt inflammation with increasing rhAC, even at 0.1 mg/kg/dose.

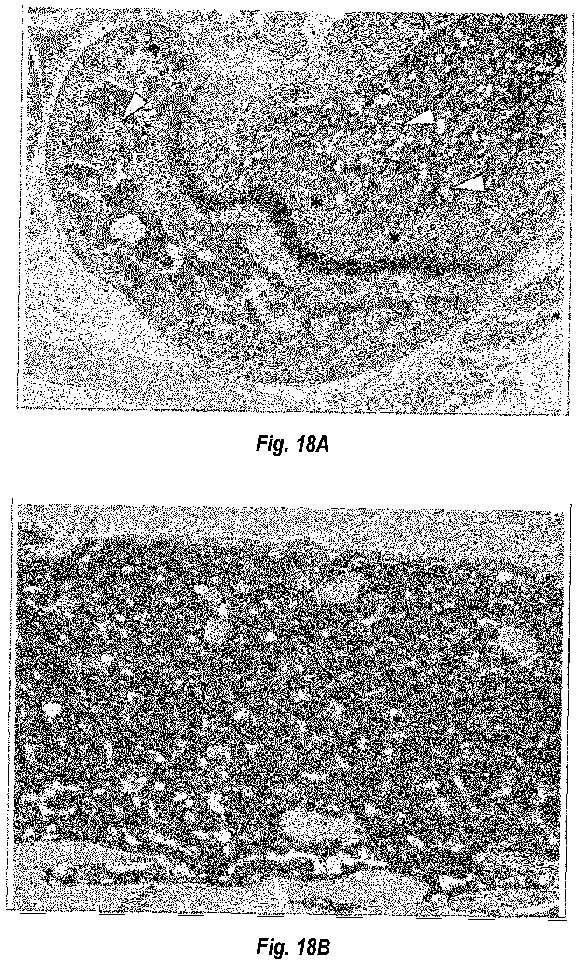

**Figs. 18A and 18B****.** The impact of rhAC on Farber mouse bone and joint pathology. Fig. 18A depicts the histology of growth plate and synovial soft tissues in a normal mouse, showing a consistent width of the physis (growth plate), robust trabeculae in diaphysis and epiphysis (white arrows), and linear organized chondrocytes within spongiosa with trabeculae radiating from the physis into the shaft marrow (asterisks). Fig. 18B depicts the histology of bone marrow in a normal mouse, showing confluent sheets of hematopoietic cells in various stages of maturation with pronounced eosinophilic staining.

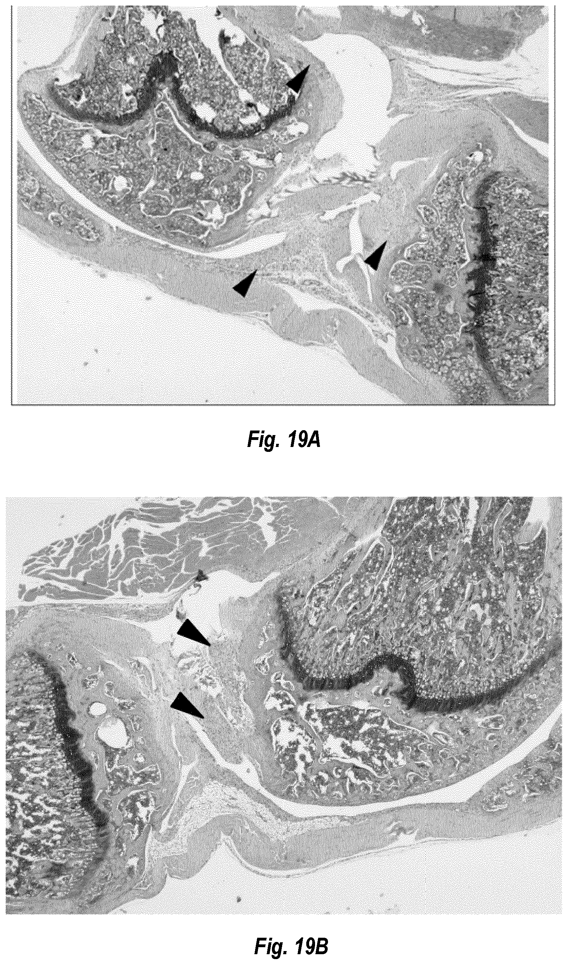

**Figs. 19A and 19B****.** The effect of rhAC on Farber mouse bone at 4x magnification. Fig. 19A depicts Farber mouse bone without rhAC, and Fig. 19B depicts Farber mouse bone after administration of rhAC at a dosage of 10 mg/kg/dose. Fig. 19B shows that administration of rhAC decreased histocytic infiltration of the synovial soft tissues (black arrowheads), ligaments and adipose pads.

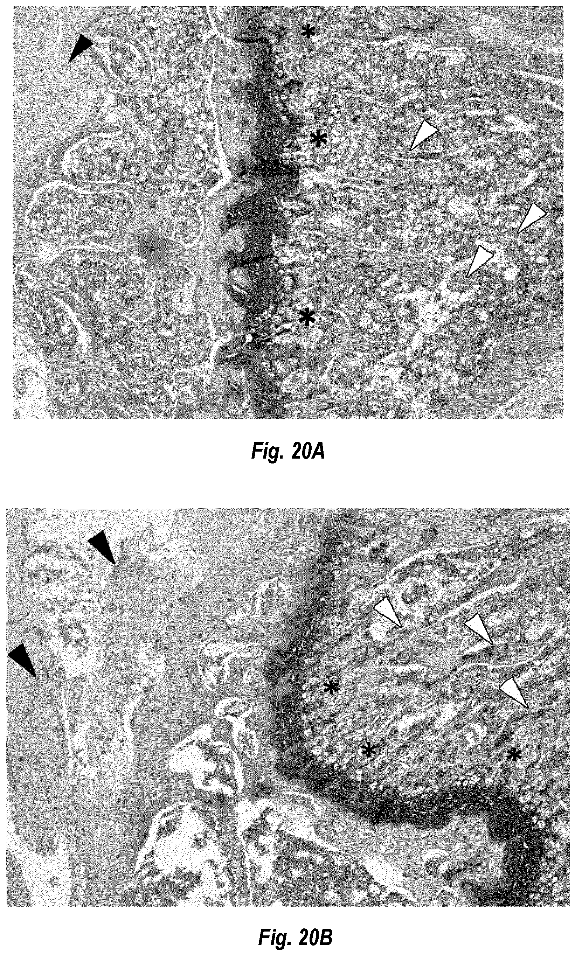

**Figs. 20A and 20B****.** The effect of rhAC on Farber mouse bone at 10x magnification. Fig. 20A depicts Farber mouse bone without rhAC, and Fig. 20B depicts Farber mouse bone after administration of rhAC at a dosage of 10 mg/kg/dose. Fig. 20B shows that administration of rhAC decreased histocytic infiltration of the synovial soft tissues (black arrowheads), ligaments and adipose pads, improved chondrocyte organization with the primary spongiosa of the physis (asterisks), showed thicker, more robust bony trabeculae in the diaphysis (white arrows), and retention of adipose pad.

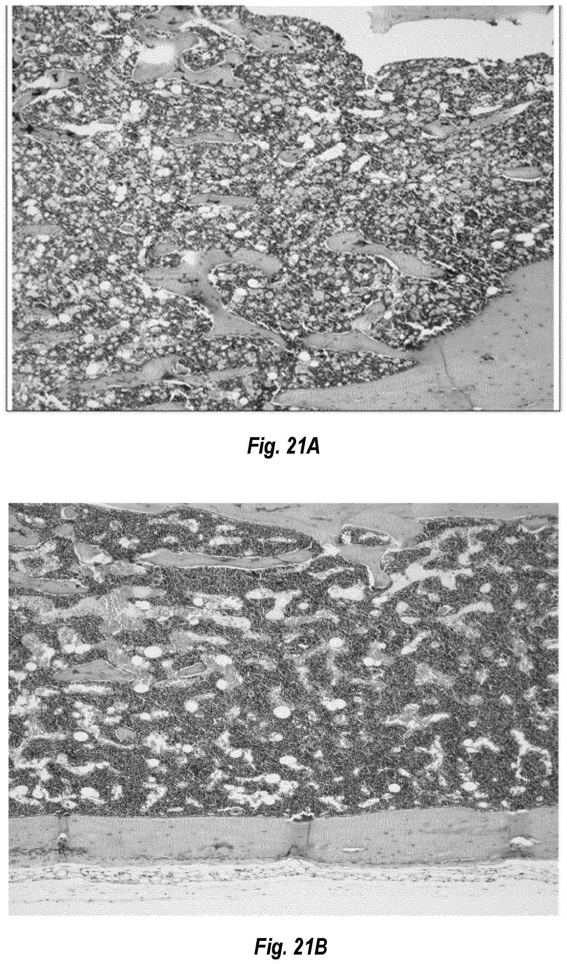

**Figs. 21A and 21B****.** The effect of rhAC on Farber mouse bone marrow at 4x magnification. Fig. 21A depicts Farber mouse bone marrow without rhAC and Fig. 21B depicts Farber mouse bone marrow after administration of rhAC at a dosage of 10 mg/kg/dose.

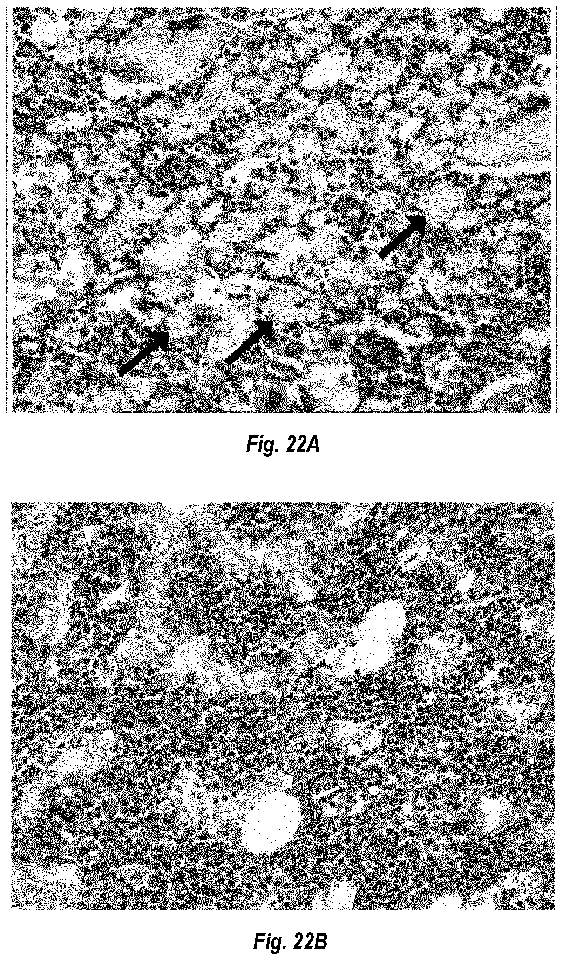

**Figs. 22A and 22B****.** The effect of rhAC on Farber mouse bone marrow at 10x magnification. Fig. 22A depicts Farber mouse bone marrow without rhAC and Fig. 22B depicts Farber mouse bone marrow after administration of rhAC at a dosage of 10 mg/kg/dose. Figs. 21B and 22B show the bone marrow of Farber mouse lacked histiocytic cellular infiltrate after administration of rhAC (comparable to a normal mouse), and normal hematopoietic cells in sheets (undisrupted).

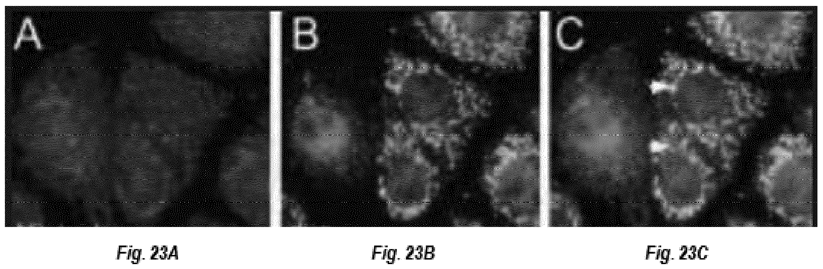

**Figs. 23A-23C****.** The effects of 10 mg/kg rhAC on macrophage polarization in tissues. Fig. 23A shows a pan-macrophage stain (F4/80). Fig. 23B shows an anti-inflammatory stain (CD206). Fig. 23C shows an overlay of Figs. 23A and 23B showing anti-inflammatory macrophages.

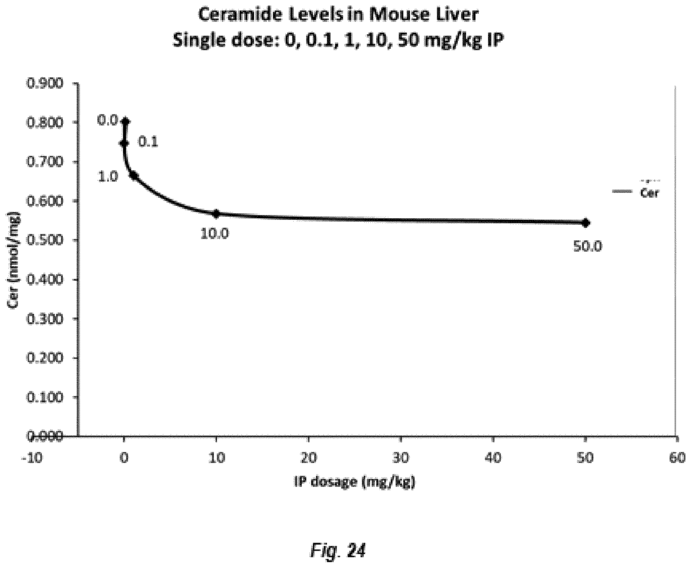

**Fig. 24****.** Ceramide concentrations in the liver of Farber disease mice after a single injection of rhAC. Groups of ~9-week-old Farber disease mice received a single intraperitoneal (IP) injection of purified rhAC at the indicated doses (n=3/dose). After 24 hours, the mice were euthanized and the amounts of total ceramide (Cer) were quantified and expressed per milligram total protein in tissue extracts from the liver. "0" indicates age-matched Farber mice that were injected with PBS. *indicates p value <0.05 comparing mice injected with rhAC to mice injected with PBS; **indicates p value <0.01.

### Detailed Description:

As used herein, the terms "a" or "an" means that "at least one" or "one or more" unless the context clearly indicates otherwise.

As used herein, the term "about" means that the numerical value is approximate and small variations would not significantly affect the practice of the disclosed embodiments. Where a numerical limitation is used, unless indicated otherwise by the context, "about" means the numerical value can vary by ±10% and remain within the scope of the disclosed embodiments.

As used herein, the term "animal" includes, but is not limited to, humans and non-human vertebrates such as wild, domestic, and farm animals. The animal can also be referred to as a "subject."

As used herein, the term "carrier" means a diluent, adjuvant, or excipient with which a compound is administered. Pharmaceutical carriers can be liquids, such as water and oils, including those of petroleum, animal, vegetable or synthetic origin, such as peanut oil, soybean oil, mineral oil, sesame oil and the like. The pharmaceutical carriers can also be saline, gum acacia, gelatin, starch paste, talc, keratin, colloidal silica, urea, and the like. In addition, auxiliary, stabilizing, thickening, lubricating and coloring agents can be used.

As used herein, the terms "comprising" (and any form of comprising, such as "comprise", "comprises", and "comprised"), "having" (and any form of having, such as "have" and "has"), "including" (and any form of including, such as "includes" and "include"), or "containing" (and any form of containing, such as "contains" and "contain"), are inclusive or open-ended and do not exclude additional, unrecited elements or method steps. Additionally, a term that is used in conjunction with the term "comprising" is also understood to be able to be used in conjunction with the term "consisting of" or "consisting essentially of."

As used herein, the term "contacting" means bringing together of two elements in an *in vitro* system or an *in vivo* system. For example, "contacting" rhAC polypeptide an individual, subject, or cell includes the administration of the polypeptide to an individual or patient, such as a human, as well as, for example, introducing a compound into a sample containing a cellular or purified preparation containing the polypeptide. Additionally, contacting can refer to transfecting or infecting a cell with a nucleic acid molecule encoding the polypeptide.

An "effective amount" of an enzyme delivered to a subject is an amount sufficient to improve the clinical course of a Farber disease where clinical improvement is measured by any of the variety of defined parameters well known to the skilled artisan.

As used herein, the terms "subject," "individual" or "patient," used interchangeably, means any animal, including mammals, such as mice, rats, other rodents, rabbits, dogs, cats, swine, cattle, sheep, horses, or primates, such as humans.

As used herein, the phrase "in need thereof' means that the subject has been identified as having a need for the particular method or treatment. In some embodiments, the identification can be by any means of diagnosis. In any of the methods and treatments described herein, the subject can be in need thereof.

As used herein, the phrase "integer from X to Y" means any integer that includes the endpoints. For example, the phrase "integer from X to Y" means 1, 2, 3, 4, or 5.

As used herein, the term "isolated" means that the compounds described herein are separated from other components of either (a) a natural source, such as a plant or cell, or (b) a synthetic organic chemical reaction mixture, such as by conventional techniques.

As used herein, the term "mammal" means a rodent (i.e., a mouse, a rat, or a guinea pig), a monkey, a cat, a dog, a cow, a horse, a pig, or a human. In some embodiments, the mammal is a human.

As used herein, the phrase "pharmaceutically acceptable" means those compounds, materials, compositions, and/or dosage forms which are, within the scope of sound medical judgment, suitable for use in contact with tissues of humans and animals. In some embodiments, "pharmaceutically acceptable" means approved by a regulatory agency of the Federal or a state government or listed in the U.S. Pharmacopeia or other generally recognized pharmacopeia for use in animals, and more particularly in humans.

As used herein, the term "purified" means that when isolated, the isolate contains at least 90%, at least 95%, at least 98%, or at least 99% of a compound described herein by weight of the isolate.

As used herein, the phrase "substantially isolated" means a compound that is at least partially or substantially separated from the environment in which it is formed or detected.

As used herein, the phrase "therapeutically effective amount" means the amount of active compound or pharmaceutical agent that elicits the biological or medicinal response that is being sought in a tissue, system, animal, individual or human by a researcher, veterinarian, medical doctor or other clinician. The therapeutic effect is dependent upon the disorder being treated or the biological effect desired. As such, the therapeutic effect can be a decrease in the severity of symptoms associated with the disorder and/or inhibition (partial or complete) of progression of the disorder, or improved treatment, healing, prevention or elimination of a disorder, or side-effects. The amount needed to elicit the therapeutic response can be determined based on the age, health, size and sex of the subject. Optimal amounts can also be determined based on monitoring of the subject's response to treatment.

Any method known to the skilled artisan may be used to monitor disease status and the effectiveness the therapy. Clinical monitors of disease status may include but are not limited to ceramide levels, weight, joint length, inflammation, or any other clinical phenotype known to be associated with Farber disease.

As used herein, the terms "treat," "treated," or "treating" mean both therapeutic treatment and prophylactic measures wherein the object is to slow down (lessen) an undesired physiological condition, disorder or disease, or obtain beneficial or desired clinical results. For example, beneficial or desired clinical results include, but are not limited to, alleviation of symptoms; diminishment of extent of condition, disorder or disease; stabilized (i.e., not worsening) state of condition, disorder or disease; delay in onset or slowing of condition, disorder or disease progression; amelioration of the condition, disorder or disease state or remission (whether partial or total), whether detectable or undetectable; an amelioration of at least one measurable physical parameter, not necessarily discernible by the patient; or enhancement or improvement of condition, disorder or disease. Thus, "treatment of Farber disease" or "treating Farber disease" means an activity that alleviates or ameliorates any of the primary phenomena or secondary symptoms associated with Farber disease or other condition described herein.

It is further appreciated that certain features described herein, which are, for clarity, described in the context of separate embodiments, can also be provided in combination in a single embodiment. Conversely, various features which are, for brevity, described in the context of a single embodiment, can also be provided separately or in any suitable subcombination.

In some embodiments, methods of treating Farber disease are provided. In some embodiments, the subject is a subject in need thereof. In some embodiments, the subject in need thereof is diagnosed with Farber disease. In some embodiments, the subject is also identified as having: 1) subcutaneous nodules; 2) an acid ceramidase activity value in white blood cells, cultured skin fibroblasts or other biological sources (e.g., plasma) that is less than 30% of control values; and/or 3) nucleotide changes within both alleles of the acid ceramidase gene (ASAH1) that indicate, through bioinformatic, gene expression studies, and/or other methods, a possible loss of function of the acid ceramidase protein. In some embodiments, the methods comprising administering to the subject a pharmaceutical composition comprising a recombinant human acid ceramidase in an effective amount of about 0.1 mg/kg to about 50 mg/kg.

In some embodiments, methods of reducing lipogranulomas in a subject with, or suspected of having, Farber disease are provided. In some embodiments, the subject is a subject in need thereof. In some embodiments, the methods comprising administering to the subject a pharmaceutical composition comprising a recombinant human acid ceramidase in an effective amount of about 0.1 mg/kg to about 50 mg/kg.

In some embodiments, methods of reducing spleen weight in a subject with, or suspected of having, Farber disease are provided. In some embodiments, the subject is a subject in need thereof. In some embodiments, the methods comprising administering to the subject a pharmaceutical composition comprising a recombinant human acid ceramidase in an effective amount of about 0.1 mg/kg to about 50 mg/kg.

In some embodiments, methods of reducing ceramide weight in a subject with, or suspected of having, Farber disease are provided. In some embodiments, the subject is a subject in need thereof. In some embodiments, the methods comprising administering to the subject a pharmaceutical composition comprising a recombinant human acid ceramidase in an effective amount of about 0.1 mg/kg to about 50 mg/kg. Reducing ceramide can also refer to decreasing ceramide or increasing the metabolizing of ceramide, which would lead to reduced ceramide levels.

In some embodiments, methods of increasing sphingosine weight in a subject with, or suspected of having, Farber disease are provided. In some embodiments, the subject is a subject in need thereof. In some embodiments, the methods comprising administering to the subject a pharmaceutical composition comprising a recombinant human acid ceramidase in an effective amount of about 0.1 mg/kg to about 50 mg/kg.

Various pharmaceutical compositions are described herein and can be used based upon the patient's and doctor's preferences. However, in some embodiments, the pharmaceutical composition is a solution. In some embodiments, the pharmaceutical composition comprises cell conditioned media comprising the rhAC. As used herein, the term "cell conditioned media" refers to cell culture media that has been used to culture cells expressing rhAC and where the protein is secreted into the media and then the protein is isolated or purified from the media. In some embodiments, the media is used to treat the subject. The media, for example, can be applied to the skin of a subject to treat any of the conditions, symptoms, or disorders described herein.

In addition to the routes of administration described herein, in some embodiments, the pharmaceutical composition is administered by contacting the skin of the subject. In some embodiments, the administration is parenteral administration. In some embodiments, the administration comprises injecting the pharmaceutical composition to the subject. In some embodiments, the administration is an intraperitoneal injection or intravenous injection.

In some embodiments, methods of treating Farber disease in a subject in need thereof are provided, wherein the method comprises expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg.

In some embodiments, methods of reducing lipogranulomas in a subject with, or suspected of having, Farber disease are provided, the methods comprising expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg.

In some embodiments, the method comprises treating lipogranulomatosis in a subject with, or suspected of having, Farber disease are provided, the methods comprising expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg.

In some embodiments, methods of reducing spleen weight in a subject with, or suspected of having, Farber disease are provided, the methods comprising expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg.

In some embodiments, methods of reducing ceramide in a subject with, or suspected of having, Farber disease are provided, the methods comprising expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg.

In some embodiments, methods of increasing sphingosine in a subject with, or suspected of having, Farber disease, the methods comprising expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg.

In some embodiments, the expressing recombinant human acid ceramidase (rhAC) in a cell comprises transferring a vector encoding rhAC into the cell. In some embodiments, the vector comprises a nucleic acid molecule encoding rhAC. In some embodiments, the nucleic acid molecule is a molecule as described herein or any other nucleic acid molecule that encodes the rhAC polypeptide or homolog thereof, which is described in more detail herein. In some embodiments, the vector is a viral vector. For example, the vector can be a retroviral vector or a DNA virus vector, such as adenovirus, AAV, and the like. In some embodiments, the vector is a plasmid. In some embodiments, the vector comprises a promoter operably linked to the rhAC. In some embodiments, the promoter is a constitutive promoter. In some embodiments, the promoter is the SV40 promoter, CMV promoter, EF 1 alpha promoter, or any combination thereof, or any other promoter that is active in a mammalian cell.

In some embodiments, the vector is transfected or infected into the cell. The methods of introducing the vector in the cell are not critical and any method can be used to provide sufficient expression of the rhAC polypeptide in the cell.

In some embodiments, the cell is a mammalian cell. In some embodiments, the cell is not a human cell. In some embodiments, the cell is a hamster cell. In some embodiments, the cell is a Chinese hamster ovarian (CHO) cell. In some embodiments, the cell can be grown in a serum-free or substantially free of serum environment. In some embodiments, the cell is derived from a CHO-K1 cell. In some embodiments, the cell is a murine cell. In some embodiments, the cell is a murine myeloma cell. In some embodiments, the cell is a NS0 cell. In some embodiments, the effective amount that is administered is as described herein, above and below.

In some embodiments, the pharmaceutical composition is administered as described herein. For example, in some embodiments, the composition is administered to a subject orally, by inhalation, by intranasal instillation, topically, transdermally, parenterally, subcutaneously, intravenous injection, intra-arterial injection, intramuscular injection, intraplurally, intraperitoneally, intrathecally, or by application to a mucous membrane.

As used herein, the term "rhAC" refers to recombinant human acid ceramidase. In some embodiments, the rhAC comprises an amino acid sequence of SEQ ID NO: 1.

In some embodiments, the rhAC is a protein that is a protein that is a homolog of SEQ ID NO: 1. In some embodiments, the rhAC is encoded by a nucleic acid molecule of SEQ ID NO: 2. In some embodiments, the rhAC is encoded by a nucleic acid molecule of SEQ ID NO: 3. In some embodiments, the rhAC is encoded by a nucleic acid molecule of SEQ ID NO: 4. In some embodiments, the sequence is as defined in GenBank accession number NM_177924.3 or NM_177924.4, each of which is incorporated by reference in its entirety. The nucleotide sequence encoding the protein can be the complete sequence shown in SEQ ID NO: 2, SEQ ID NO: 3, or SEQ ID NO: 4, or be simply the coding region of the sequence The coding region, for example, could be nucleotides 313 to 1500 of SEQ ID NO: 2 or the corresponding coding region found in SEQ ID NO: 3 or SEQ ID NO: 4. However, as is well known to one of skill in the art, the genetic code is degenerate and, therefore other codons can be used to encode the same protein without being outside of what is disclosed. Since the amino acid sequence is known any nucleotide sequence that encodes the amino acid sequence is acceptable. In some embodiments, the nucleotide sequence comprises a signal peptide. In some embodiments, the signal peptide is an amino acid sequence encoded by nucleotides 313 to 375 of SEQ ID NO: 2. In some embodiments, the protein that is produced comprises a signal peptide of amino acid residues 1-21 of SEQ ID NO: 1. In some embodiments, the protein that is produced does not comprises a signal peptide, such as the signal peptide of amino acid residues 1-21 of SEQ ID NO: 1. In some embodiments, the signal peptide is removed during a post-translational process where the enzyme is processed into its different subunits. In some embodiments, the nucleotide sequence is codon optimized for the cell that it the protein is being expressed from. In some embodiments, the protein comprises an alpha-subunit, a beta-subunit, and the like. In some embodiments, the protein that is produced comprises a peptide of amino acid residues 22-142, 45-139, 134-379, 143-395, or 1-395 of SEQ ID NO: 1. The peptide can be a single protein or a polypeptide of different sequences to form the enzyme. In some embodiments, the protein is free of amino acid residues 1-21. These regions can be encoded by a single nucleotide sequence or separate nucleotide sequences or a combination of nucleotide sequences. As discussed herein, any nucleotide sequence encoding the protein can be used and is not limited to the sequence described herein as SEQ ID NO: 2, SEQ ID NO: 3, or SEQ ID NO: 4.

In some embodiments, the rhAC has acid ceramidase (AC) activity but does not have any detectable acid sphingomyelinase activity, such as the rhAC produced in Examples 1 and 2 below. The acid sphingomyelinase activity may be removed, for example, by heat inactivation. See, e.g., U.S. Patent Application Publication No. 20160038574, which is incorporated herein in its entirety. Heat inactivation may also remove other contaminating proteins from an rhAC preparation. *Id.*

**Table 1.**

| SEQ ID NO | Sequence |
|---|---|
| 1 | |
| 2 | GGCTCGGTCCGACTATTGCCCGCGGTGGGGGAGGGGGATGGATCAC GCCACGCGCCAAAGGCGATCGCGACTCTCCTTCTGCAGGTAGCCTG GAAGGCTCTCTCTCTTTCTCTACGCCACCCTTTTCGTGGCACTGAAA AGCCCCGTCCTCTCCTCCCAGTCCCGCCTCCTCCGAGCGTTCCCCCT ACTGCCTGGAATGGTGCGGTCCCAGGTCGCGGGTCACGCGGCGGAG GGGGCGTGGCCTGCCCCCGGCCCAGCCGGCTCTTCTTTGCCTCTGCT GGAGTCCGGGGAGTGGCGTTGGCTGCTAGAGCGATGCCGGGCCGG AGTTGCGTCGCCTTAGTCCTCCTGGCTGCCGCCGTCAGCTGTGCCGT CGCGCAGCACGCGCCGCCGTGGACAGAGGACTGCAGAAAATCAAC CTATCCTCCTTCAGGACCAACGTACAGAGGTGCAGTTCCATGGTAC ACCATAAATCTTGACTTACCACCCTACAAAAGATGGCATGAATTGA TGCTTGACAAGGCACCAGTGCTAAAGGTTATAGTGAATTCTCTGAA GAATATGATAAATACATTCGTGCCAAGTGGAAAAATTATGCAGGTG |
| | GTGGATGAAAAATTGCCTGGCCTACTTGGCAACTTTCCTGGCCCTTT TGAAGAGGAAATGAAGGGTATTGCCGCTGTTACTGATATACCTTTA GGAGAGATTATTTCATTCAATATTTTTTATGAATTATTTACCATTTGT ACTTCAATAGTAGCAGAAGACAAAAAAGGTCATCTAATACATGGG AGAAACATGGATTTTGGAGTATTTCTTGGGTGGAACATAAATAATG ATACCTGGGTCATAACTGAGCAACTAAAACCTTTAACAGTGAATTT GGATTTCCAAAGAAACAACAAAACTGTCTTCAAGGCTTCAAGCTTT GCTGGCTATGTGGGCATGTTAACAGGATTCAAACCAGGACTGTTCA GTCTTACACTGAATGAACGTTTCAGTATAAATGGTGGTTATCTGGGT ATTCTAGAATGGATTCTGGGAAAGAAAGATGTCATGTGGATAGGGT TCCTCACTAGAACAGTTCTGGAAAATAGCACAAGTTATGAAGAAGC CAAGAATTTATTGACCAAGACCAAGATATTGGCCCCAGCCTACTTT ATCCTGGGAGGCAACCAGTCTGGGGAAGGTTGTGTGATTACACGAG ACAGAAAGGAATCATTGGATGTATATGAACTCGATGCTAAGCAGGG TAGATGGTATGTGGTACAAACAAATTATGACCGTTGGAAACATCCC TTCTTCCTTGATGATCGCAGAACGCCTGCAAAGATGTGTCTGAACC GCACCAGCCAAGAGAATATCTCATTTGAAACCATGTATGATGTCCT GTCAACAAAACCTGTCCTCAACAAGCTGACCGTATACACAACCTTG ATAGATGTTACCAAAGGTCAATTCGAAACTTACCTGCGGGACTGCC CTGACCCTTGTATAGGTTGGTGAGCACACGTCTGGCCTACAGAATG CGGCCTCTGAGACATGAAGACACCATCTCCATGTGACCGAACACTG CAGCTGTCTGACCTTCCAAAGACTAAGACTCGCGGCAGGTTCTCTTT GAGTCAATAGCTTGTCTTCGTCCATCTGTTGACAAATGACAGATCTT TTTTTTTTCCCCCTATCAGTTGATTTTTCTTATTTACAGATAACTTCT TTAGGGGAAGTAAAACAGTCATCTAGAATTCACTGAGTTTTGTTTC ACTTTGACATTTGGGGATCTGGTGGGCAGTCGAACCATGGTGAACT CCACCTCCGTGGAATAAATGGAGATTCAGCGTGGGTGTTGAATCCA GCACGTCTGTGTGAGTAACGGGACAGTAAACACTCCACATTCTTCA GTTTTTCACTTCTACCTACATATTTGTATGTTTTTCTGTATAACAGCC TTTTCCTTCTGGTTCTAACTGCTGTTAAAATTAATATATCATTATCTT TGCTGTTATTGACAGCGATATAATTTTATTACATATGATTAGAGGGA TGAGACAGACATTCACCTGTATATTTCTTTTAATGGGCACAAAATG GGCCCTTGCCTCTAAATAGCACTTTTTGGGGTTCAAGAAGTAATCA GTATGCAAAGCAATCTTTTATACAATAATTGAAGTGTTCCCTTTTTC ATAATTACTCTACTTCCCAGTAACCCTAAGGAAGTTGCTAACTTAAA |
| | AAACTGCATCCCACGTTCTGTTAATTTAGTAAATAAACAAGTCAAA GACTTGTGGAAAATAGGAAGTGAACCCATATTTTAAATTCTCATAA GTAGCATTCATGTAATAAACAGGTTTTTAGTTTGTTCTTCAGATTGA TAGGGAGTTTTAAAGAAATTTTAGTAGTTACTAAAATTATGTTACTG TATTTTTCAGAAATCAAACTGCTTATGAAAAGTACTAATAGAACTT GTTAACCTTTCTAACCTTCACGATTAACTGTGAAATGTACGTCATTT GTGCAAGACCGTTTGTCCACTTCATTTTGTATAATCACAGTTGTGTT CCTGACACTCAATAAACAGTCACTGGAAAGAGTGCCAGTCAGCAGT CATGCACGCTGATTGGGTGTGT |
| 3 | AAGCTTACCGCCACCATGAACTGCTGCATCGGCCTGGGTGAGAAGG CGCGTGGCTCGCACCGCGCCAGCTACCCCTCCCTGAGCGCCCTCTTC ACCGAGGCGTCCATCCTCGGATTCGGGAGCTTCGCCGTCAAGGCAC AGTGGACCGAGGATTGCCGCAAGAGTACGTACCCCCCCAGTGGCCC GACGTACCGCGGCGCCGTCCCCTGGTACACGATCAACCTGGACCTC CCCCCGTACAAGCGCTGGCACGAGTTGATGCTGGACAAGGCCCCCG TACTGAAGGTCATCGTGAACTCCCTGAAGAACATGATCAACACCTT CGTCCCCTCGGGCAAGATCATGCAGGTCGTGGACGAGAAGCTGCCC GGGCTCCTCGGCAACTTCCCCGGCCCGTTCGAAGAGGAGATGAAGG GCATCGCGGCCGTCACTGACATCCCCCTGGGCGAGATCATCAGCTT CAACATCTTCTACGAGCTGTTCACCATCTGCACCTCCATCGTAGCCG AGGACAAGAAGGGCCACCTGATCCACGGTCGCAACATGGACTTCG GCGTCTTCCTGGGCTGGAACATCAACAACGACACCTGGGTCATCAC CGAGCAGCTGAAGCCGCTCACCGTGAACCTCGATTTCCAGCGCAAC AACAAGACGGTGTTCAAGGCCAGCTCCTTCGCCGGGTACGTCGGGA TGCTCACGGGCTTCAAGCCGGGACTGTTCTCGCTGACCCTCAACGA GCGGTTCTCCATCAACGGGGGCTACCTCGGCATCCTGGAGTGGATT CTCGGCAAGAAGGACGTGATGTGGATCGGCTTCCTCACACGGACCG TGCTGGAAAACTCCACTAGTTACGAGGAGGCCAAGAACCTGCTGAC CAAGACGAAGATCCTGGCCCCGGCATACTTCATCCTGGGCGGCAAC CAGTCGGGCGAGGGGTGCGTCATCACCCGCGACCGGAAGGAGTCC CTGGACGTCTATGAGCTGGACGCCAAGCAGGGCCGCTGGTACGTCG TCCAGACGAACTACGACCGATGGAAGCACCCCTTCTTCCTCGACGA CCGGCGCACGCCCGCCAAGATGTGCCTGAACCGCACCAGCCAGGA GAACATCTCGTTCGAGACGATGTACGACGTGCTGTCGACCAAGCCC |
| | GTGCTCAACAAGCTGACGGTCTACACCACGCTGATCGACGTGACGA AAGGCCAGTTCGAAACGTACCTGCGGGACTGCCCGGACCCTTGCAT CGGCTGGTGATAATCTAGAGTCGGGGCGGCCGGCC |
| 4 | AAGCTTACCGCCACCATGAACTGCTGCATCGGGCTGGGAGAGAAAG CTCGCGGGTCCCACCGGGCCTCCTACCCAAGTCTCAGCGCGCTTTTC ACCGAGGCCTCAATTCTGGGATTTGGCAGCTTTGCTGTGAAAGCCC AATGGACAGAGGACTGCAGAAAATCAACCTATCCTCCTTCAGGACC AACGTACAGAGGTGCAGTTCCATGGTACACCATAAATCTTGACTTA CCACCCTACAAAAGATGGCATGAATTGATGCTTGACAAGGCACCAG TGCTAAAGGTTATAGTGAATTCTCTGAAGAATATGATAAATACATT CGTGCCAAGTGGAAAAATTATGCAGGTGGTGGATGAAAAATTGCCT GGCCTACTTGGCAACTTTCCTGGCCCTTTTGAAGAGGAAATGAAGG GTATTGCCGCTGTTACTGATATACCTTTAGGAGAGATTATTTCATTC AATATTTTTTATGAATTATTTACCATTTGTACTTCAATAGTAGCAGA AGACAAAAAAGGTCATCTAATACATGGGAGAAACATGGATTTTGG AGTATTTCTTGGGTGGAACATAAATAATGATACCTGGGTCATAACT GAGCAACTAAAACCTTTAACAGTGAATTTGGATTTCCAAAGAAACA ACAAAACTGTCTTCAAGGCTTCCAGCTTTGCTGGCTATGTGGGCATG TTAACAGGATTCAAACCAGGACTGTTCAGTCTTACACTGAATGAAC GTTTCAGTATAAATGGTGGTTATCTGGGTATTCTAGAATGGATTCTG GGAAAGAAAGATGTCATGTGGATAGGGTTCCTCACTAGAACAGTTC TGGAAAATAGCACAAGTTATGAAGAAGCCAAGAATTTATTGACCAA GACCAAGATATTGGCCCCAGCCTACTTTATCCTGGGAGGCAACCAG TCTGGGGAAGGTTGTGTGATTACACGAGACAGAAAGGAATCATTGG ATGTATATGAACTCGATGCTAAGCAGGGTAGATGGTATGTGGTACA AACAAATTATGACCGTTGGAAACATCCCTTCTTCCTTGATGATCGCA GAACGCCTGCAAAGATGTGTCTGAACCGCACCAGCCAAGAGAATAT CTCATTTGAAACCATGTATGATGTCCTGTCAACAAAACCTGTCCTCA ACAAGCTGACCGTATACACAACCTTGATAGATGTTACCAAAGGTCA ATTCGAAACTTACCTGCGGGACTGCCCTGACCCTTGTATAGGTTGGT GATAACCTAGGGTCGGGGCGGCCGGCC |

The term "homolog" refers to protein sequences having between 80% and 100% sequence identity to a reference sequence. Percent identity between two peptide chains can be determined by pair wise alignment using the default settings of the AlignX module of Vector NTI v.9.0.0 (Invitrogen Corp., Carslbad, Calif.). In some embodiments, the homolog has at least, or about, 80, 85, 86, 87, 88, 89, 90, 91, 92, 93, 94, 95, 96, 97, 98, or 99% identity to a sequence described herein, such as SEQ ID NO: 1. In some embodiments, the protein delivered to the subject conservative substitutions as compared to a sequence described herein. Non-limiting exemplary conservative substitutions are shown in Table 2 are encompassed within the scope of the disclosed subject matter. Substitutions may also be made to improve function of the enzyme, for example stability or enzyme activity. Conservative substitutions will produce molecules having functional and chemical characteristics similar to those molecules into which such modifications are made. Exemplary amino acid substitutions are shown in Table 2 below.

**Table 2: Exemplary Conservative Substitutions:**

| Original Residue | Exemplary Conservative Substitutions |
|---|---|
| Ala | Val, Leu, Ile |
| Arg | Lys, Gln, Asn |
| Asn | Gln |
| Asp | Glu |
| Cys | Ser, Ala |
| Gln | Asn |
| Gly | Pro, Ala |
| His | Asn, Gln, Lys, Arg |
| Ile | Leu, Val, Met, Ala, Phe |
| Leu | Ile, Val, Met, Ala, Phe |
| Lys | Arg, Gln, Asn |
| Met | Leu, Phe, Ile |
| Phe | Leu, Val, Ile, Ala, Tyr |
| Pro | Ala |
| Ser | Thr, Ala, Cys |
| Thr | Ser |
| Trp | Tyr, Phe |
| Tyr | Trp, Phe, Thr, Ser |
| Val | Ile, Met, Leu, Phe, Ala |

The term "in combination with" as used herein means that the described agents can be administered to a subject together in a mixture, concurrently as single agents or sequentially as single agents in any order.

As described herein, in some embodiments, the protein is produced from a cell. In some embodiments, the cell is a Chinese Hamster Ovarian cell, "CHO cell." A nucleic acid sequence encoding the proteins described herein can be genomic DNA or cDNA, or RNA (*e.g.* mRNA) which encodes at least one of proteins described herein. The use of cDNA requires that gene expression elements appropriate for the host cell be combined with the gene in order to achieve synthesis of the desired protein. The use of cDNA sequences can advantageous over genomic sequences (which contain introns), in that cDNA sequences can be expressed in bacteria or other hosts which lack appropriate RNA splicing systems. One of skill in the art can determine the best system for expressing the protein.

In some embodiments, the protein is produced according to U.S. Patent Application Publication No. 20160038574, which is incorporated by reference in its entirety.

Because the genetic code is degenerate, more than one codon can be used to encode a particular amino acid. Using the genetic code, one or more different oligonucleotides can be identified, each of which would be capable of encoding the amino acid sequences described herein.

The enzyme that is administered to the subject to treat Farber disease or a condition associate therewith can be purified. The term "purified" with referenced to a protein refers to a protein that is substantially free of other material that associates with the molecule in its natural environment. For instance, a purified protein is substantially free of the cellular material or other proteins from the cell or tissue from which it is derived. The term refers to preparations where the isolated protein is sufficiently pure to be analyzed, or at least 70% to 80% (w/w) pure, at least 80%-90% (w/w) pure, 90-95% pure; and, at least 95%, 96%, 97%, 98%, 99%, or 100% (w/w) pure. In some embodiments, the protein is purified from a cell, such as but not limited to a CHO cell.

### Administration, Compositions, and Kits Comprising the Proteins

As described herein, embodiments provided herein provide methods of treating Farber disease. In some embodiments, the methods comprise administering a therapeutically or prophylactically effective amount of one or more proteins described herein to a subject with Farber disease or suspected of having Farber disease.

Treatment of subjects may comprise the administration of a therapeutically effective amount of the proteins described herein.

The proteins can be provided in a kit as described herein.

The proteins can be used or administered alone or in admixture with an additional therapeutic. Examples of additional therapeutics include, but are not limited to, inhibitors of acid sphingomyelinase (e.g., amitryptiline (Becker et al., "Acid Sphingomyelinase Inhibitors Normalize Pulmonary Ceramide and Inflammation in Cystic Fibrosis," Am. J. Respir. Cell. Mol. Biol., 42:716-724 (2010), which is hereby incorporated by reference in its entirety) and inhibitors of ceramide synthases (e.g., Shiffmann et al., "Inhibitors of Specific Ceramide Synthases," Biochimie, 94:558-565 (2012), which is hereby incorporated by reference in its entirety)). The additional therapeutic can also be ceramidase mixtures described in U.S. Patent Application Publication No. 20160038574, which is hereby incorporated by reference in its entirety.

While enzyme replacement therapies (ERTs) can be effective, as shown in our current study for Farber disease where reduction of AC accumulation was demonstrated, antibodies can develop against the drug, i.e., the replacement enzyme that may reduce its efficacy. Here, we have shown that repeat dosages are well tolerated, which supports a treatment regimen of repeated administration of the replacement enzyme resulting in reduction of the symptoms of the disease, particularly the enzyme that is produced according to the methods described herein and, e.g., in U.S. Patent Application Publication No. 20160038574.

In some embodiments, methods of treating Farber disease in a subject in need thereof comprise administering to the subject a pharmaceutical composition comprising a recombinant human acid ceramidase in an effective amount about once a week, once every 2, 3, or 4 weeks, or once a month, for about 10, about 20, or about 30 weeks, 1, 5, 10, or 25 years, or the duration of a patient's life.

Suitable vehicles and their formulation and packaging are described, for example, in Remington: The Science and Practice of Pharmacy (21st ed., Troy, D. ed., Lippincott Williams & Wilkins, Baltimore, Md. (2005) Chapters 40 and 41). Additional pharmaceutical methods may be employed to control the duration of action. Controlled release preparations may be achieved through the use of polymers to complex or absorb the compounds. Another possible method to control the duration of action by controlled release preparations is to incorporate the compounds of into particles of a polymeric material such as polyesters, polyamino acids, hydrogels, poly(lactic acid) or ethylene vinylacetate copolymers. Alternatively, instead of incorporating these agents into polymeric particles, it is possible to entrap these materials in microcapsules prepared, for example, interfacial polymerization, for example, hydroxymethylcellulose or gelatin-microcapsules and poly(methylmethacylate)-microcapsules, respectively, or in colloidal drug delivery systems, for example, liposomes, albumin microspheres, microemulsions, nanoparticles, and nanocapsules or in macroemulsions.

In general, if administering a systemic dose of the protein, it is desirable to provide the recipient with a dosage of protein which is in the range of from about 1 ng/kg-100 ng/kg, 100 ng/kg-500 ng/kg, 500 ng/kg-1 ug/kg, 1 ug/kg-100 ug/kg, 100 ug/kg-500 ug/kg, 500 ug/kg-1 mg/kg, 1 mg/kg-50 mg/kg, 50 mg/kg-100 mg/kg, 100 mg/kg-500 mg/kg (body weight of recipient), although a lower or higher dosage may be administered.

In some embodiments, the effective amount of rhAC that is administered is about 0.1 mg/kg to about 10 mg/kg. In some embodiments, the effective amount is about 10 mg/kg to about 50 mg/kg. In some embodiments, the effective amount is about 10 mg/kg to about 20 mg/kg. In some embodiments, the effective amount is about 20 mg/kg to about 30 mg/kg. In some embodiments, the effective amount is about 30 mg/kg to about 40 mg/kg. In some embodiments, the effective amount is about 40 mg/kg to about 50 mg/kg. In some embodiments, the effective amount is about 1, 2, 3, 4, 5, 6, 7, 8, 9, or 10 mg/kg.

The dosage can be administered once a day, twice a day, three times a day, four times a day, once a week, twice a week, once every two weeks, or once a month. In some embodiments, the dose is administered once a week. The treatment may also be given in a single dose schedule, or a multiple dose schedule in which a primary course of treatment may be with 1-10 separate doses, followed by other doses given at subsequent time intervals required to maintain and or reinforce the response, for example, once a week for 1-4 months for a second dose, and if needed, a subsequent dose(s) after several months. Examples of suitable treatment schedules include: (i) 0, 1 month and 6 months, (ii) 0, 7 days and 1 month, (iii) 0 and 1 month, (iv) 0 and 6 months, or other schedules sufficient to elicit the desired responses expected to reduce disease symptoms, or reduce severity of disease. Other treatment schedules, such as, but not limited to, those described above, can also be used.

In certain aspects of the invention, the treatment is started when the subject is under 1, 2, 3, 4, 5, 6, 7, 8, 9, or 10 years of age, or between 1 and 2, 3, 4, 5, 6, 7, 8, 9, 10, 25, 50, 60, 70 or 80 years of age (e.g., between 1 and 2, between 1 and 3, etc.). In some embodiments, the subject is between 16 and 61. In some embodiments, the subject starts treatment at age 16. In some embodiments, the subject is between 12 and 69. In some embodiments, the subject starts treatment at age 12. In some embodiments, the subject is between 19 and 74. In some embodiments, the subject starts treatment at age 19. In some embodiments, the subject is between 4 and 62. In some embodiments, the subject starts treatment at age 4. In some embodiments, the subject is between 7 and 42. In some embodiments, the subject starts treatment at age 7. In some embodiments, the subject is between 1 and 6 months. In some embodiments, the subject starts treatment at age 1 month, 2 months, 3 months, 4 months, 5 months, or 6 months. In some embodiments, the subject is between 6 and 43. In some embodiments, the subject starts treatment at age 6. In some embodiments, the subject is between 5 and 31. In some embodiments, the subject starts treatment at age 5. In some embodiments, the subject is between 5 and 57. In some embodiments, the subject is between 5 and 29. In some embodiments, the subject is between 1 and 3. In some embodiments, the subject starts treatment at age 1. In some embodiments, the subject is between 10 and 70. In some embodiments, the subject starts treatment at age 10. In some embodiments, the subject is between 5 and 80, between 10 and 70, between 20 and 75, between 5 and 60, or between 5 and 30 years of age.

In some embodiments, a subject diagnosed with Farber disease is administered rhAC at about 1 mg/kg to about 5 mg/kg rhAC or about 2 mg/kg to about 5 mg/kg rhAC every two weeks. In one embodiment, the dosage escalates from 1 mg/kg or 2 mg/kg to 5 mg/kg at week 4. If a dose level is not tolerated by an individual subject, the dose for that subject may be reduced from 2 mg/kg to 1 mg/kg, or 5 mg/kg to 2 mg/kg, as appropriate. The rhAC may be administered every 2 weeks for at least 10, 20, or 30 weeks or for the duration of the subject's life. In one embodiment, a subject is diagnosed with Farber disease and is identified as having: 1) subcutaneous nodules; and/or 2) an acid ceramidase activity value in white blood cells, cultured skin fibroblasts or other biological sources (e.g., plasma) that is less than 30% of control values; and/or 3) nucleotide changes within both alleles of the acid ceramidase gene (ASAH1) that indicate, through bioinformatic, gene expression studies, and/or other methods, a possible loss of function of the acid ceramidase protein. In some embodiments, the subject is administered rhAC every two weeks for 28 weeks. In some embodiments, the delivery of rhAC is by intravenous infusion (e.g., saline infusion). In some embodiments, starting at about 2 mg/kg and escalating to about 5 mg/kg rhAC (e.g., to 5 mg/kg at week 4).

For example, site specific administration may be to body compartment or cavity such as intrarticular, intrabronchial, intraabdominal, intracapsular, intracartilaginous, intracavitary, intracelial, intracelebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, intralesional, vaginal, rectal, buccal, sublingual, intranasal, or transdermal means.

The therapeutic compositions described herein can be prepared for use for parenteral (subcutaneous, intramuscular or intravenous) or any other administration particularly in the form of liquid solutions or suspensions. The formulation can also be suitable for an injectable formulation. In some embodiments, the injectable formulation is sterile. In some embodiments, the injectable formulation is pyrogen free. In some embodiments, the formulation is free of other antibodies that bind to other antigens other than an antigen described herein.

A protein of rhAC capable of treating Farber disease or other condition associated with rhAC activity or use to treat a rhAC related pathology, is intended to be provided to subjects in an amount sufficient to affect a reduction, resolution, or amelioration in the related symptom or pathology. Such a pathology, includes the symptoms of Farber disease as described herein in a subject. An amount is said to be sufficient or a "therapeutically effective amount" to "affect" the reduction of symptoms if the dosage, route of administration, and dosing schedule of the agent are sufficient to influence such a response. Responses to the protein can be measured by analysis of subject's affected tissues, organs, or cells as by imaging techniques or by *ex vivo* analysis of tissue samples. An agent is physiologically significant if its presence results in a detectable change in the physiology of a recipient patient. In some embodiments, an amount is a therapeutically effective amount if it is an amount that can be used to treat, ameliorate or inhibit symptoms of Farber disease that a subject is subject to. Non-limiting examples of such amounts are provided herein, but are not intended to be limited to such amount if context dictates another amount.

In some embodiments, efficacy of treatment is assessed by any of the following means:
- Percent change from baseline in net nodule (≥5 mm) count after treatment with rhAC for 28 weeks;
- Percent change from baseline in net nodule (≥10 mm) count and comparison to placebo after treatment with rhAC for 28 weeks;
- Percent change from baseline in total nodule count (regardless of size) and comparison to placebo after treatment with rhAC for 28 weeks;
- Change and percent change from baseline of joint range of motion in selected joints and comparison to placebo after treatment with rhAC for 28 weeks;
- Change and percent change from baseline of 6 minute walk distance and comparison to placebo after treatment with rhAC for 28 weeks;
- Change and percent change from baseline of pulmonary function tests and comparison to placebo after treatment with rhAC for 28 weeks;
- Change and percent change from baseline of FDT score and comparison to placebo after treatment with rhAC for 28 weeks;
- Change and percent change from baseline in Z score of body weight and height for age during treatment with rhAC or placebo over 28 weeks.

The proteins can be formulated according to known methods to prepare pharmaceutically useful compositions, whereby these materials, or their functional derivatives, are combined in admixture with a pharmaceutically acceptable carrier vehicle.

Kits, which are described herein and below, are also provided which are useful for carrying out embodiments described herein. In some embodiments, the kits comprise a first container containing or packaged in association with the above-described polypeptides. The kit may also comprise another container containing or packaged in association solutions necessary or convenient for carrying out the embodiments. The containers can be made of glass, plastic or foil and can be a vial, bottle, pouch, tube, bag, etc. The kit may also contain written information, such as procedures for carrying out the embodiments or analytical information, such as the amount of reagent contained in the first container means. The container may be in another container apparatus, e.g. a box or a bag, along with the written information.

Yet another aspect provided for herein is a kit for treating Farber disease. In some embodiments, the kit comprises at least one container comprising a rhAC polypeptide or a nucleic acid molecule encoding the same. In some embodiments, the kit comprises a container comprising a cell that is configured to express rhAC. In some embodiments, the cell is a CHO cell. In some embodiments, the kit comprises conditioned media from a cell that expresses rhAC. In some embodiments, the conditioned media is from a CHO cell.

The subject matter is now described with reference to the following examples. These examples are provided for the purpose of illustration only and the claims should in no way be construed as being limited to these examples, but rather should be construed to encompass any and all variations which become evident as a result of the teaching provided herein. Those of skill in the art will readily recognize a variety of non-critical parameters that could be changed or modified to yield essentially similar results.

### Examples

Farber disease was first described in 1952 in a 14-month-old infant with granulomatous lesions on multiple joints and evidence of lipid storage (Farber, 1952). Over the ensuing decade other similar cases were described, all of whom had similar lesions and often exhibited a characteristic "hoarse" cry or voice due to the presence of lesions on the larynx. The involvement of other organ systems in some of these patients, including the lung, liver, spleen and central nervous system (CNS), also was noted.

In 1972 Sugita et al. (Sugita et al., 1972) demonstrated that post-mortem tissues and cells from several Farber disease patients exhibited acid ceramidase (AC) deficiency. Acid ceramidase (E.C. #3.5.1.23) is a lipid hydrolase first identified in 1963 (Gatt). The enzyme had a pH optimum of ~5, suggesting that it was a component of the lysosomal system, although it was also shown that at physiologic pH it could carry out a "reverse" reaction in which ceramides where synthesized using fatty acids and sphingosine as substrates (for review see Schuchman et al., 2016).

The first substantial purification of the enzyme was in 1995 from human urine (Bernardo et al., 1995). The purified urinary enzyme was an ~50 kDa polypeptide that could be reduced into 13 and 40 kDa polypeptides (a- and b-subunits, respectively). Deglycosylation studies revealed the presence of 5 or 6 N-glycosylation sites on the b-subunit. The availability of highly purified recombinant human AC (rhAC) led to the isolation of the cDNA and gene encoding AC (ASAH1) (Koch et al., 1996; Li et al., 1999), the identification of the first mutations causing Farber disease (Koch et al., 1999), and the production and purification of rhAC from Chinese hamster ovary (CHO) cells (He et al., 2003).

Characterization of rhAC purified from CHO cell media showed that it was composed of three polypeptides associated by disulfide bonds; a precursor polypeptide and the a and β-subunits. The precursor polypeptide was inactive until an internal cleavage at cysteine residue 142 resulted in the subunit formation. Further studies showed that AC was a self-cleaving enzyme and underwent "auto-activation" (Shtraizent et al., 2008). Farber disease cells could internalize rhAC, leading to the degradation of the accumulating ceramides. The recombinant enzyme also was able to carry out the reverse reaction, although the physiological significance of this reaction and the factors that govern the balance between the degradative and synthetic functions remain unknown (Okino et al., 2003).

Prior to the present disclosure, treatment for Farber disease patients has been symptomatic and principally aimed at reducing pain. Hematopoietic stem cell transplantation (HSCT) has been undertaken in several patients (e.g., Torcoletti et al., 2014), and overall the outcome has been positive provided that the transplant procedure itself was successful. Such transplanted patients exhibit significant reduction in pain, increased motility and mobility, and in some cases shrinking and complete resolution of the subcutaneous nodules. However, successful transplantation requires histocompatible donor cells, and exposes patients to invasive and potentially dangerous immunosuppressant regimes. One alternative to HSCT is gene therapy in which autologous donor cells are transduced with a vector expressing the therapeutic protein, obviating the need for histocompatible donors. This approach has been evaluated in the Farber disease knock-in mice and resulted in reduction of tissue ceramides and macrophage infiltration (Alayoubi et al., 2013).

The present Example demonstrates that enzyme replacement therapy (ERT) for treating Farber disease is efficacious based upon the use of ERT in a knock-in mouse model using rhAC. Without being bound to any particular theory, we believe that the results provided herein demonstrate that rhAC treatment will reverse and/or prevent the ceramide-driven inflammatory response that is responsible for the infiltration of lipid-filled macrophages into tissues of Farber mice and patients, particularly at cartilage sites, and that reduction of ceramide leads to better outcomes and treatment.

### Example 1

### Materials and Methods

### Production & Characterization of rhAC

A human AC cDNA (derived from NM_177924.3; ASAH1 variant 1) was introduced into the Selexis SURE CHO-M cell line^{™} (Selexis SA, Switzerland), and clones overexpressing AC activity were selected. One overexpressing clone (MST-cp07-cp47) was further expanded and grown in a bioreactor system (GE Healthcare Life Sciences Inc.). After filtration rhAC was purified from the media by sequential ion exchange and size fractionation chromatography. Prior to its use in animals the *in vitro* physical and biochemical characteristics (e.g., pH optimum, isoelectric point, molecular weight, subunit association) were compared to the previously described CHO-derived rhAC (He et al., 2003) by established methods. The rhAC produced was determined to have no detectable acid sphingomyelinase activity.

### Farber Mouse Colony

A colony of *asah1*^{P361R/P361R} mutant mice (i.e., Farber disease mice) were maintained on a mixed genetic background (W4/sv129/C57Bl) by breeding heterozygous mating pairs as previously described (Alayoubi et al., 2013). Genotyping was carried out by analysis of toe clip DNA at weaning (3 weeks) unless otherwise noted. All experiments were performed at the Icahn School of Medicine under a protocol (#98-0089) approved by the Institutional Animal Care and Use Committee. Wild-type mice were used as controls and derived from within the colony. Animals were fed *ad libitum* food and water and euthanasia was performed by ketamine/xylazine injections followed by cervical dislocation according to NIH guidelines.

### Cell Culture Analysis

Dr. Thierry Levade (Toulouse, France) kindly provided a previously characterized (Chatelut et al., 1997) EBV-transformed fibroblast cell line from a Farber disease patient. Cells were grown in RPMI culture media (Sigma-Aldrich) containing 10% heat inactivated fetal bovine serum (FBS) (v/v), 1% penicillin/streptomycin (v/v), 1% L-glutamine (v/v) and 0.1% fungizone (v/v). To evaluate rhAC secreted into the MST-cp07-cp47 media, conditioned media was collected from the CHO cell clone grown in shaker flasks for 48 h. The transformed Farber disease fibroblasts were grown to -80% confluency and the standard RPMI medium was exchanged for the conditioned medium. Cells were then grown for an additional 48 h in the absence of serum, after which they were trypsinized and harvested with a rubber policeman. The cell pellets were washed 3X with PBS, and lipid assays were performed on cell lysates as described below.

Chondrocytes were isolated from the articular surfaces of mouse menisci. The tissue isolates were collected in fresh DMEM (Thermo Fisher) containing 10% FBS (v/v), 1% penicillin/streptomycin (v/v), 1% L-glutamine (v/v) and 0.1% fungizone (v/v), minced with scissors, and then transferred to DMEM containing 1 mg/mL protease and rotated at 37°C for 2 h. They were then incubated in DMEM containing 1 mg/mL collagenase type II and rotated at 37°C overnight. The remaining tissue was strained three times through 40 µm nylon mesh filters to remove debris. The cell suspension was centrifuged (1000 rpm for 5 minutes) and cells were plated at a density of 10,000 cells/cm² and cultured in complete DMEM. Media was changed every 3 days. For rhAC supplementation experiments, cells were grown with (12.5 µg/ml) and without rhAC in the medium. rhAC was only added on day 1, when the cells were first plated. Subsequent media changes did not include rhAC.

### rhAC Preparation & Enzyme Administration

Purified rhAC obtained from the media of the MST-cp07-cp47 CHO cell clone was maintained at a concentration of 10 mg/ml in sterile PBS and stored at -20°C. It was subjected to only one thaw cycle prior to use. Enzyme administration into Farber mice was by intraperitoneal (i.p.) injection unless otherwise noted. The amount of enzyme administered to mice was based on the desired dose (in µg/g) and the weight of the animals. If necessary, the enzyme was diluted in sterile PBS prior to administration. Control Farber disease mice were injected with PBS alone.

### Quantification of Total Tissue Ceramides & Sphingosine

Lipid extracts were prepared from tissue homogenates or cell lysates by the classic Folch method (Folch et al., 1957) using chloroform/methanol (2:1). The lipid extract was then dried under nitrogen gas and re-dissolved in a 2% Igepal solution. For ceramide determination, a ceramide hydrolysis buffer (0.2 M citric/phosphate buffer, pH 4.5 containing 0.3 M NaCl and 0.2 µg/µl of rhAC) was mixed with the total lipid extract solution (1:1, v/v) and incubated at 37°C for 60 min. For our standard reaction, 2 µl each of lipid extract and ceramide hydrolysis buffer was used. This mixture was then incubated for an additional 10 min at 50°C with 56 µl of a fluorogenic reaction buffer (25 mM sodium borate buffer, pH 9) containing 1.25 mM sodium cyanide and 1.25 mM naphthalene-2,3-dicarboxyaldehyde (NDA) to derivatize the sphingosine.

The mixture was then centrifuged (13,000xg/10 min) and 5 µl of the supernatant was analyzed using an Acquity H-Class UPLC system (Waters) equipped with a Waters Acquity UPLC BEH RP18 column (2.0×50 mm, 1.7 micrometers). The mobile phase composition for the gradient system was 0.1% ammonium hydroxide for mobile phase A, and 100% acetonitrile for mobile phase B. The gradient program was 0-0.01 min 36-4% A, 64-96% B, 0.01-0.3 min 4-36% A, 96-64% B, 0.3-1 min 36% A, 64% B at a flow rate of 1 ml/min. The fluorescent (NDA) sphingosine was monitored at excitation and emission wavelengths of 252 and 483 nm, respectively. Quantification of the sphingosine peak was calculated using the Waters Empower software according to a standard curve derived from commercial (Molecular Probes) NDA sphingosine.

For quantification of sphingosine the same procedure was used except that the ceramide hydrolysis step was excluded. In this way, endogenous sphingosine present in the lipid extract could be derivatized directly with NDA.

### AC Activity Assay

Samples (tissue homogenates or cell lysates) were incubated at 37°C (1:1, v/v) with substrate buffer (0.2 mM NBD-C12 ceramide, 0.2 M citrate/phosphate buffer, pH 4.5, 0.3 M NaCl, 10% FBS, and 0.2% Igepal) for 30 min. NBD-C12 ceramide was purchased from Avanti Polar Lipids. The reaction was stopped by ethanol (10x) and centrifuged (13,000xg/10 min), and the supernatant (5 µl) was analyzed using the Acquity H-Class UPLC system (Waters). Separation of the undegraded NBD-C12 ceramide substrate and NBD-C12 fatty acid reaction product was achieved using a Waters Acquity UPLC BEH C18 column (2.0×30 mm, 1.7 µm). The mobile phase composition for the gradient system was 13 mM ammonium acetate buffer (pH, 7.2) for mobile phase A and 100% acetonitrile for mobile phase B. The gradient program was 0-0.1 min 68-0% A, 32-100% B, 0.1-0.4 min 0-68% A, 100-32% B, 0.4-0.8 min 68% A, 32% B at a flow rate of 1.2 ml/min. The fluorescent product (NBD-C12 fatty acid) was monitored at excitation and emission wavelengths of 435 nm and 525 nm, respectively. Quantification of the product peak was calculated using the Waters Empower software according to a standard curve derived from commercial NBD-C12 fatty acid (Avanti).

### MCP-1 ELISA

Immediately following euthanasia, blood was collected from mice by heart puncture and plasma was frozen at -20°C. Plasma monocyte chemoattractant protein (MCP)-1 was determined by ELISA using a commercial kit (#MJE00, R & D Systems) according to a protocol supplied by the manufacturer.

### RT-qPCR Analysis

After 7 or 14 days of expansion, chondrocytes with and without rhAC supplementation were harvested from the culture flasks (~1 × 10⁶ cells/pellet). RNA was extracted using the qiaShredder and RNeasy Mini Kit (Qiagen, Limburg, Netherlands) and quantified using the Nanodrop 1000 (Thermo Scientific, Walthman, MA). Complementary DNA was synthesized using the same amount of RNA from each group using the high capacity cDNA reverse transcription kit (Life Technologies, Grand Island, NY) and a Bio-Rad S1000 thermal cycler (Bio-Rad, Hercules, CA). RT-qPCR was completed using the fast, universal PCR Master Mix and primers (Life Technologies, Grand Island, NY) specific for collagen II (Col2a1, Rn01637087_m1), aggrecan (Agg, Rn00573424_m1), Sox9 (Sox9, Rn01751069_mH), and GAPDH (Rn01775763_g1 as a housekeeping gene), and ran on a 7900HT qPCR machine (Life Technologies, Grand Island, NY). The ΔΔct method was used to analyze the data and the results were presented as relative quantity (RQ) fold increase.

### Histopathology

Following euthanasia, the tissues were harvested from mice and fixed in 10% formalin for 24 h, and then stored in ethanol until ready for analysis. For H&E staining they were paraffin embedded and sectioned (5 µ) with a microtome.

### Statistics

Comparisons between two groups were performed with a Student's t-test. In cases where more than two groups were compared to each other, a one-way analysis of variance (ANOVA) was used, followed by a Tukey's HSD test. All statistical analyses were performed using SPSS statistical software.

### Results

### In Vitro Assessment of rhAC

The rhAC used for this study was purified from the media of an overexpressing CHO cell clone (MST-cp07-cp47). As shown in Fig. 11, the enzyme was highly purified and exhibited a ~50 kDa band under non-reducing SDS PAGE conditions and ~13 and 40 kDa bands under reducing conditions, corresponding to the expected α- and β-subunits, respectively. In order to confirm the biological activity of this secreted enzyme, conditioned media was collected from the MST-cp07-cp47 cells and added to EBV transformed fibroblasts obtained from a Farber disease patient (Chatelut et al., 1997). After 24 h the cells were harvested and the total ceramides and sphingosine were quantified. Fig. 1A and Fig. 1B illustrate that cells grown in the conditioned media (CM) had significantly reduced ceramides (Fig. 1A) and elevated sphingosine (Fig. 1B) compared to cells grown in standard media (M), demonstrating that the secreted rhAC could be internalized by cells and was catalytically active.

### Acute Dosing of Farber Disease Mice with rhAC

Initial studies in the Farber mice evaluated single administration of purified rhAC into ~9-week-old animals at 4 different doses (0.1, 1, 10 and 50 mg/kg). At this age affected mice exhibit massive ceramide storage in tissues (Alayoubi et al., 2013). Intraperitoneal (i.p.) injections were used rather than tail vein due to the extremely small size of the mice and relatively large volume (~75 µl) required for the high dose (50 mg/kg) injection.

Figs. 2A-2D and Fig. 24 show the effect of the different rhAC doses on total ceramides (Cer) (Fig. 2A and Fig. 24) and sphingosine (Sph) (Fig. 2B) in the livers and spleens (Figs. 2C and 2D, respectively) of the Farber disease mice at 24 h post-injection. Overall, rhAC administration led to ceramide reductions in both tissues, albeit the maximal reductions were only ~40%, likely due to the massive ceramide build-up and the fact that only a single administration of rhAC was used. Benefit was seen with a dose of 1 mg/kg. Similar benefit was also seen with higher doses of 10 mg/kg and 50 mg/kg.

In contrast to ceramide reduction, a dose-responsive increase in sphingosine was observed in both tissues. Importantly, no adverse reactions to the enzyme injections were evident, including at the high dose of 50 mg/kg.

To further evaluate the pharmacokinetics of the enzyme *in vivo,* Farber disease mice received a single rhAC injection of 10 mg/kg, and AC activity, total ceramides, and sphingosine were determined in the livers (Figs. 3A, 3B, and 3C, respectively) and spleens (Figs. 3D, 3E, and 3F, respectively) at 12, 24, 48, 72, and 168 h post-injection. AC activity in the liver peaked at 12 h post-injection, and was maintained for up to 48 h. By 72 h the liver AC activity returned to baseline. Liver ceramides were maximally reduced by 24 h and remained at these low levels for up to 168 hr. Sphingosine levels were increased at 12 h post-injection (corresponding with ceramide breakdown), but returned to baseline by 24 h. This indicated either rapid degradation or metabolism of the rhAC-derived sphingosine.

A similar pharmacokinetic pattern was observed in spleen, except that the peak AC activity in the spleen was slightly delayed compared to the liver (peak AC activity at 24 vs. 12 h). We also evaluated the plasma half-life of AC activity in Farber disease mice following single tail vein injection, and found that the T_{1/2} was ~36 min (Fig. 12).

Based on the above dosing studies, we concluded that rhAC was bioactive *in vivo* (reduced ceramide and produced sphingosine), and that single i.p. injections into Farber disease mice could be well tolerated at doses up to 50 mg/kg. We also concluded that repeat dosing of at least once per week should be sufficient to maintain low ceramides and achieve therapeutic effects due to the relatively slow rate of re-accumulation after enzyme administration.

### Repeat Dosing of Farber Disease Mice With rhAC

Next, groups of Farber disease mice were injected i.p. with three doses of rhAC (1, 3 and 10 mg/kg), once per week beginning at ~3 weeks of age. Mice were maintained on enzyme treatment until they required euthanasia according to IACUC protocol (loss of >10% body weight within 1 week). Thus, the primary endpoint for this study was survival.

Fig. 4 illustrates a modest benefit of enzyme injection on survival, evident in all treatment groups. However, no dose response was observed. Modest effects of ERT on prevention of weight loss also were found, but again no clear dose response was noted (Fig. 13). When the mice reached their euthanasia endpoint, blood and tissues were collected for analysis. Note that since survival was the primary endpoint for this study, not all animals received the same number of enzyme injections prior to euthanasia. Euthanasia was carried out within 2-3 days of the last enzyme injection.

ERT reduced spleen weight in the Farber disease mice to that of normal mice, even at the 1 mg/kg dose (Fig. 5A). Plasma MCP-1, a macrophage chemokine that is elevated in Farber disease mice and patients (Dworski et al., 2017), also was significantly reduced by ERT, although the levels never reached those of normal animals (Fig. 5B).

Total ceramides and sphingosine were quantified in six tissues (liver, spleen, kidney, lung, heart and brain) after euthanasia. Dose responsive reduction of ceramides by ERT was observed in all tissues (Figs. 6A-6F). Liver ceramides were reduced to normal (Fig. 6A), spleen ceramides were reduced by >80% (Fig. 6C), and heart (Fig. 6B), lung (Fig. 6F) and kidney (Fig. 6D) ceramides by >60%. Surprisingly, brain ceramides were modestly reduced compared to untreated Farber disease mice at doses up to 3 mg/kg (Fig. 6E).

Of interest, we also found that sphingosine was substantially elevated in several Farber disease mouse tissues (liver (Fig. 7A), kidney (Fig. 7D) and brain (Fig. 7E). We assume that the elevation of sphingosine in these tissues was due to breakdown of accumulating ceramides by non-lysosomal ceramidases. Following rhAC administration, sphingosine levels were reduced to normal by ERT in the liver (Fig. 7A) and brain (Fig. 7E), and also significantly reduced in the kidney (Fig. 7D). A clear dose response was observed.

Histological assessments also were carried out on representative liver and spleen sections from treated and control Farber disease mice, and reductions of storage macrophages were observed following ERT (Figs. 8A and 8B). All Farber disease mice tolerated the repeat enzyme injections well and no drug-related adverse events were noted.

Overall, the above findings demonstrated that ERT in Farber disease mice had a significant impact on several important endpoints, including tissue ceramides and sphingosine, macrophage infiltration, spleen size, and plasma MCP-1 levels. Surprisingly, there also was some evidence that rhAC might influence the brain (e.g., reduction of accumulating sphingosine).

To further explore these findings, we next carried out a study in which Farber disease mice were treated at a dose of 10 mg/kg (once weekly, i.p.), starting at ~3 days of age, immediately after genotyping. The rationale underlying this study was that early treatment might provide additional benefit in the brain, as in other mouse LSD studies (Vogler et al., 1999).

As shown in Fig. 14, early treatment provided a significant survival benefit (mean survival of 101 days vs. 78 days). Lipid analysis (Figs. 9A-9F) further showed that ceramides in liver (Fig. 9A), spleen (Fig. 9C), heart (Fig. 9B), and lung (Fig. 9F) also were reduced in animals undergoing early treatment as compared to those started at 3 weeks. Surprisingly, however, early treatment resulted in higher ceramides in kidney (Fig. 9D) and brain (Fig. 9E). It should be noted that mice subjected to early treatment received on average ~6-7 more rhAC injections than those started at 3 weeks due to the early starting age and longer survival period. Sphingosine also was significantly reduced in the livers with early treatment (Fig. 15A), and modestly reduced in the lung (Fig. 15F). The levels in other organs were comparable between the two treatment groups (Figs. 15B heart, 15C spleen, 15D kidney, and Fig. 15E brain).

### In Vitro Assessment of rhAC Treatment on Farber Disease Mouse Chondrocytes

As noted above, macrophage-filled nodules ("lipogranulomas") form at cartilage sites in all or most Farber disease patients, leading to debilitating cartilage disease. Farber disease mice do not form visible nodules, and thus this feature of the human disorder could not be evaluated in these ERT studies. However, we isolated chondrocytes from the affected mice and treated them with rhAC *in vitro* to assess enzyme uptake and efficacy by this important cell type. As shown in Figs. 10A-10C, chondrocytes from untreated Farber disease mice exhibited very low expression of several chondrogenic marker genes (collagen 2 (Fig. 10A), aggrecan (Fig. 10B), and Sox-9 (Fig. 10C)), and addition of rhAC to the culture media markedly enhanced expression. These findings demonstrated uptake of the enzyme by this important pathologic cell type and the potential of correcting the chondrogenic phenotype.

### Discussion

The goal of these experiments was to use the mouse model of Farber disease to establish proof-of-concept for future ERT studies in Farber disease patients. The Farber disease mouse is a knock-in of a severe human Farber disease mutation (P362R), and correspondingly the mice exhibit a severe disease that results in death between -7-13 weeks of age. Affected mice are born exceptionally small and exhibit failure to thrive and a rapid decline beginning at ~4-5 weeks of age. As in patients, they accumulate ceramides in most tissues and exhibit macrophage infiltration. They also have high levels of several inflammatory biomarkers, of which MCP-1 is the most significant and consistent. Unlike patients, Farber disease mice do not develop visible nodules at cartilage sites, although there is histological evidence of macrophage infiltration in cartilage and synovial tissues. The fact that they do not develop nodules might be due to their markedly shortened lifespan.

The rhAC used for these studies was purified from the media of overexpressing CHO cells. The cell line expressed the same cDNA as in our previous work (He et al., 2003), except that it was prepared and maintained under "good manufacturing practice" (GMP) conditions. In addition, the purification protocol was modified from our previous work to accommodate scale-up and eventual clinical use. As shown in Fig. 11, this rhAC exhibited the same molecular weight and subunit association as our previous rhAC. To confirm the bioactivity of this enzyme, conditioned media from the overexpressing CHO cells was used to treat SV40-transformed Farber disease skin fibroblasts, and resulted in significant ceramide reduction and sphingosine elevation compared to cells grown in standard tissue culture media.

Acute injection studies using this rhAC in the Farber disease mice further revealed the bioactivity of this enzyme, resulting in a reduction of tissue ceramides and production of sphingosine. Several important findings were observed from these acute dosing studies: a) doses as low as 0.1 mg/kg had biological effect, b) doses up to 50 mg/kg were well tolerated and exhibited no toxic effect, c) there was no advantage of the 50 mg/kg dose compared to 10 mg/kg, d) sphingosine elevation exhibited a more linear and dose responsive pattern than ceramide reduction, and e) even in 9-week-old animals with considerable disease, reversal of ceramide storage was evident. It was also notable that after a single injection of rhAC ceramide levels remained reduced for up to 1 week, indicating a relatively slow re-accumulation rate.

Several interesting points arise from these observations. At the outset, we predicted that we might observe some toxicity to high dose rhAC administration in Farber disease mice, in part due to the production of sphingosine, a highly toxic and bioactive sphingolipid, particularly in the liver where the majority of the rhAC is delivered. A similar high dose toxicity has been observed in acid sphingomyelinase deficient (Niemann-Pick disease) mice, and was attributed to the production of ceramide, which is subsequently converted to sphingosine (Murray et al., 2015). We did not observe this in the Farber disease mice, perhaps due to the relatively low levels of sphingosine produced and the transient nature of the elevation. In fact, although nanomole levels of sphingosine should have been produced in tissues from ceramide hydrolysis by rhAC, only picomole levels were detected (see Figs. 2A-2D and Figs. 3A-3F). This indicated that the majority of the sphingosine produced by rhAC was either rapidly degraded or re-metabolized. In the future, it will be of interest to examine the baseline levels of enzymes involved in sphingosine metabolism in Farber disease mice and patients, including sphingosine kinases and sphingosine lysase, to see if overexpression of these enzymes could be responsible for this observation. The current data also warrant a more detailed lipidomic analysis to identify additional lipids that might be produced by rhAC treatment and represent downstream sphingosine metabolites. Also, acute dosing studies in the Farber disease mice revealed a relatively slow re-accumulation of ceramide after enzyme administration, suggesting that this ERT can likely be administered to Farber disease patients in the clinic every other week, or perhaps less frequently. It is also of interest that we observed a sustained and somewhat biphasic expression of AC activity in the liver following rhAC administration (up to 48 h, Fig. 3A). This could be due to initial delivery, secretion and then re-uptake by liver cells, allowing for more sustained release.

We next evaluated repeat administration of the enzyme in the Farber disease mice. Treatment was started at ~3 weeks (immediately after weaning) since even at this young age affected mice exhibit considerable pathology. We considered this design clinically relevant since Farber disease patients commonly go through a period of delayed diagnosis, and are therefore likely to exhibit established disease before treatment is initiated. Due to their very small size and difficulty injecting the tail vein, we chose to use i.p. injections for more consistent dosing. However, in the clinic it is likely that rhAC will be administered by i.v. infusion. Work in other LSD animal models has shown that following i.p. injections in mice, recombinant enzymes exhibit a delayed but sustained release into the blood, after which they follow a similar pharmacology to tail vein injections (Bae et al., 2004).

The liver is the major site of enzyme uptake for both i.v. and i.p. injections. Correspondingly, in our studies liver exhibited the most consistent response to rhAC administration. Liver ceramides, which are massively elevated in the Farber disease mice, were reduced to normal with rhAC treatment (Fig. 6A). The lowest dose evaluated (1 mg/kg) exhibited a >80% reduction in liver ceramides. Spleen, heart, kidney and lung each exhibited significant ceramide reductions as well (Figs. 6C, 6B, 6D, and 6F, respectively). Surprisingly, even brain exhibited some ceramide reduction at the 1 and 3 mg/kg doses, although at 10 mg/kg the levels were similar to untreated Farber mice (Fig. 6E).

It is also notable that three tissues in the Farber disease mice (brain, liver and kidney) exhibited significant sphingosine storage, and that rhAC treatment resulted in depletion of sphingosine from each of these tissues (Figs. 7E, 7A, and 7D, respectively). The origin of sphingosine storage in Farber disease remains unknown, but could be due to progressive degradation of accumulating ceramides that distribute from lysosomes to other cellular compartments where they are exposed to other ceramidases.

It is important to recognize that the primary endpoint in these repeat administration experiments was survival, and that we therefore treated the Farber disease mice until they exhibited a weight loss that required euthanasia according to our IACUC protocol. Thus, not all animals received the same number of injections, even if they received the same dose. This could contribute to variability of the data within and between dose groups. Overall, we did observe a modest survival benefit to rhAC administration (on average ~10-days), although there was no clear dose response (Fig. 4).

Of interest, when we started rhAC administration (10 mg/kg) at an earlier age (~3 days vs. 3 weeks), the survival benefit was significantly extended (Fig. 14). Studies in other lysosomal storage disease (LSD) mouse models have shown that such early enzyme administration may facilitate delivery to the brain due to the delayed closing of the blood brain barrier (Vogler et al., 1999), and we hypothesize that this might be the case here. Although the cause of death has not been established in the Farber disease mice, there is substantial brain pathology in this model that could be contributing to the early death. However, ceramide was not substantially reduced in the brain by early treatment, and in fact was even elevated (as in the kidney). The mechanism underlying this finding remains unknown.

In the future, these dose-responsive lipid changes should be further evaluated. For example, the question of why some tissues demonstrated higher ceramide levels at the higher doses or when enzyme administration was started at a very early age requires further attention, as does its relationship to the enhanced survival. That said, the data presented here clearly show elevation of tissue ceramides and sphingosine in the Farber disease mice, and that repeat administration of rhAC led to significant reductions of these lipids in most tissues.

We also followed spleen weight in the treated mice, and noted a very significant reduction in response to rhAC administration (Fig. 5A). Even at the 1 mg/kg dose the spleen size was reduced to near normal. We similarly determined the levels of MCP-1 in the plasma of the treated mice. MCP-1 is a macrophage chemokine that is significantly elevated in the blood of Farber disease mice and patients (Alayoubi et al., 2013), and we observed a significant reduction in MCP-1 in the mice receiving rhAC administration (Fig. 5B). In animals receiving 1 mg/kg, MCP-1 was reduced ~50%, although no clear dose response was observed in the 3 and 10 mg/kg groups and the levels never reached those of normal animals. Consistent with the reduction in MCP-1, we also observed reduced macrophage infiltration into Farber disease mouse liver and spleen following rhAC administration.

Thus, from the repeat administration studies, we conclude that rhAC a) can be safely administered to Farber disease mice, b) is bioactive and results in significant ceramide and sphingosine changes in tissues, c) results in a small survival advantage that can be improved by early administration, d) normalizes spleen size, e) significantly reduces plasma MCP-1, and f) reduces macrophage infiltration into tissues.

Lastly, although this Farber disease mouse model does not develop visible nodules at cartilage sites, we did isolate primary chondrocytes and show for the first time that there was very low expression of several genes essential for chondro differentiation (aggrecan, collagen 2, sox-9). Addition of rhAC to the culture media could correct this phenotype, further indicating the bioactivity of the enzyme and the fact that it can be taken up by this important cell type. We and others have shown that there is an important and unique relationship between sphingolipids and cartilage integrity (Simonaro et al., 2013, Frohbergh et al., 2016), and in the future, it will be interesting to examine this further in the context of Farber disease.

Overall, we conclude that these proof-of-concept studies support the further development of ERT for this disorder. rhAC administration resulted in significant correction of the tissue lipid profiles and also significantly reduced at least one important inflammatory cytokine (MCP-1), as well as the infiltration of inflammatory cells into tissues. Spleen weight also was normalized and there was a modest effect on lifespan. Although the effect of ERT on cartilage nodules could not be evaluated in this model, given the clear effect of the enzyme on reducing the inflammatory profile it is expected that the nodules will be reduced significantly in patients receiving ERT since they are composed primarily of lipid-filled macrophages that have infiltrated into this tissue.

### Example 2

### Methods

### rhAC Drug Supply and Preparation

rhAC was purified from the reactor media of an rhAC-overexpressing CHO-M clonal cell line (MST-cp07-cp47) (He et al., 2003), under cGMP conditions at GE Healthcare Life Sciences, Inc. (Marlborough, MA). rhAC bulk drug substance (Batch EN753-01-15-001) was provided as a solution in sterile phosphate buffered saline (PBS) at a concentration of 9.91 mg/mL, and diluted for injection using 0.9% sterile saline. Sterile PBS served as the vehicle control.

### Farber Mice

Farber disease knock-in mice that are homozygous for the *asah1*^{P161R/P361R} mutation, were derived from a mixed genetic background colony (W4/129Sv/CD1) as previously described (Alayoubi et al., 2013). Wild-type littermates (*asah1*^{WT/WT}) served as healthy controls. Genetic confirmation of Farber homozygous or WT homozygous littermates occurred at 3 weeks of age, just prior to weaning. All in-life experiments were approved by the Icahn School of Medicine's Institutional Animal Care and Use Committee under protocol #98-0089. Mice received standard food and water *ad libitum,* and were euthanized according to current NIH guidelines.

### Dosing Protocol

Farber mice were assigned to one of six treatment groups on a rolling basis as their genotype was confirmed, with a total of 8 animals per group. Farber mice were dosed with 0 (PBS, disease control), 0.1, 1, 3 or 10 mg/kg rhAC by intraperitoneal (i.p.) injection once weekly for 6 weeks, beginning at approximately 3 weeks of age (6 doses total). Wild-type mice treated with PBS alone served as healthy controls. 48 hours following the final dose, animals were euthanized, terminal blood was collected and processed to plasma for cytokine analysis, and the following tissues were divided equally with half being snap-frozen for tissue lipid analysis and the other half being fixed in formalin for histopathological processing: liver, spleen, lung, brain, kidney, trachea, femur (in-tact femoro-tibial joint), heart, muscle, and thymus. End of study body weight and the weight of each isolated tissue were recorded for each study animal.

### Experimental Protocols

### Quantification of Total Tissue Ceramide and Sphingosine

Half of each tissue collected 48 hours following the final dose of rhAC was snap-frozen and stored at -20°C until use. Quantification of total ceramide and sphingosine from snap-frozen tissues collected 48 hours after the sixth and final dose of rhAC was carried out as previously described (He et al., 2017). Total ceramide was quantified indirectly by hydrolyzing ceramide to sphingosine, derivatizing the sphingosine hydrolysate, and quantifying the derivatized sphingosine product. Briefly, tissues were homogenized and extracted using chloroform/methanol (2:1, v/v) by the classic Folch method (Folch et al., 1957), the lipid extract was then dried under nitrogen gas, and reconstituted in a 2% Igepal solution (Sigma-Aldrich). Total ceramide in each sample was hydrolyzed to sphingosine by diluting 2 µL of each sample 1: 1 (v/v) with a 0.2 M citric/phosphate buffer containing 0.3 M NaCl and 0.2 mg/mL rhAC (pH 4.5), and incubated for 1 hour at 37°C. 56 µL of a fluorogenic buffer containing 25 mM sodium borate, 1.25 mM sodium cyanide and 1.25 mM naphthalene-2,3-dicarboxyaldehyde (pH 9) was added and the mixture was incubated for 10 min at 50°C to derivatize the sphingosine hydrolysate. Indirect quantification of total ceramide (derivatized ceramide hydrolysate) was achieved using a Waters Acquity UPLC fitted with an RP18 column (20x50mm, 1.7 micrometers), and detected using excitation and emission wavelengths of 252 and 483 nm, respectively (previously described by He et al., 2017). Sphingosine quantification followed the same derivatization protocol, without the use of hydrolysis buffer.

Total ceramide is presented as nmol/mg tissue protein, and sphingosine is presented as pmol/mg tissue protein.

### Plasma MCP-1 Analysis

Terminal blood collected 48 hours following the final dose of rhAC and immediately following euthanasia was processed to plasma using routine methods and stored at - 20°C until use. Plasma monocyte chemoattractant protein (MCP)-1 was quantified by ELISA using a commercially kit (#MJE00, R & D Systems) according to the manufacturer's protocol. Plasma MCP-1 is presented as pg/mL plasma.

### Histopathology

Half of each tissue collected 48 hours following the final dose of rhAC was fixed in formalin and stored in 10% ethanol at room temperature until use. Tissues required for microscopic evaluation were trimmed, processed routinely, embedded in paraffin, and stained with hematoxylin and eosin by Charles River Laboratories, Inc., Durham. Microscopic evaluation was conducted by a board-certified veterinary pathologist, on all protocol-specified tissues from all animals in Groups 1 through 6 (Group 1 = WT controls, Groups 2-6 = Farber mice treated with 0 (disease control), 0.1, 1, 3 or 10 mg/kg rhAC, respectively). Treatment groups were not blinded. All available tissues were evaluated by light microscopy. When available for examination, the tissues included six sections of the brain, and one section each of the lung, trachea, esophagus, thyroid gland, skeletal muscle, thymus, heart, spleen, liver, kidney, and femoro-tibial joint from each study animal.

Pathology findings were graded using a five-grade semi-quantitative scale: minimal, mild, moderate, marked, and severe. Minimal was less than 10% of section area affected (or volume change for diffuse growth changes). Similarly, mild was 10-20%, moderate was 20-40%, marked was 40-70%, and severe was greater than 70%. Pathology grading was collected in table format using Microsoft Office Excel (2010).

### Results

Farber mice were evaluated after treatment with rhAC (rhAC) for six weeks at four different doses (0.1, 1, 3, and 10 mg/kg/dose) starting at ~3 weeks of age. Figs. 16A-16G show the effect of the different rhAC doses on total ceramides (Cer) in the liver, heart, muscle, spleen, lung, kidney, and brain, respectively, of the Farber disease mice at 24 h post-injection of the 6th dose. Overall, rhAC administration led to prevention of tissue ceramide accumulation in multiple tissues, particularly in the liver, spleen, kidney and heart. Effects were inconclusive in the lung, brain and skeletal muscle, though some drug related changes were seen with 0.1 mg/kg/dose.

Plasma monocyte chemoattractant protein (MCP)-1 was quantified in terminal blood collected 48 hours after the final dose in treated and control Farber disease mice. Quantification of MCP-1, a systemic marker of inflammation, showed a clear dose response of a transition away from overt inflammation with increasing rhAC, even at 0.1 mg/kg/dose (Fig. 17).

Histopathological assessment of tissues was also carried out on half of each tissue collected at 48 hours following the final dose of rhAC. The histiocytic infiltration was robust and multifocal in the liver, spleen, thymus, lung, soft tissue surrounding the trachea and esophagus, connective tissue of the skeletal muscle, and white matter (forebrain, midbrain, and hindbrain). Cardiac muscle and kidneys were largely unaffected by the disease state. The dose-dependent effects of rhAC were present in the liver and spleen pathology. No effects were detected on the brain, lung, thymus, esophagus, and heart. Increasing doses of rhAC at 0.1, 1, 3, and 10 mg/kg/dose (in 6 weekly doses) may contribute to decreasing incidence and/or severity of lesions in the liver, spleen, blood vessels of the liver, bone marrow, and to a lesser extent skeletal muscle and synovial soft tissues. Effects of rhAC were not detected with Farber mouse lesions in the brain, lung, trachea, thyroid, esophagus, thymus or heart.

The impact of rhAC on Farber mouse bone and joint pathology was also evaluated. Figs. 18A depicts the histology of growth plate and synovial soft tissues in a normal mouse, showing a consistent width of the physis (growth plate), robust trabeculae in diaphysis and epiphysis (white arrows), and linear organized chondrocytes within spongiosa with trabeculae radiating from the physis into the shaft marrow (asterisks). Fig. 19A depicts Farber mouse bone at 4x magnification and Fig. 19B depicts Farber mouse bone after administration of rhAC at a dosage of 10 mg/kg/dose at 4x magnification. Fig. 20A depicts Farber mouse bone at 10x magnification and Fig. 20B depicts Farber mouse bone after administration of rhAC at a dosage of 10 mg/kg/dose at 10x magnification. Surprisingly, administration of rhAC decreased histocytic infiltration of the synovial soft tissues (black arrowheads), ligaments and adipose pads, improved chondrocyte organization with the primary spongiosa of the physis (asterisks), showed thicker, more robust bony trabeculae in the diaphysis (white arrows), and retention of adipose pad.

Fig. 18B depicts the histology of bone marrow in a normal mouse, showing confluent sheets of hematopoietic cells in various stages of maturation with pronounced eosinophilic staining. Fig. 21A depicts Farber mouse bone marrow at 4x magnification and Fig. 21B depicts Farber mouse bone marrow after administration of rhAC at a dosage of 10 mg/kg/dose at 4x magnification. Fig. 22A depicts Farber mouse bone marrow at 10x magnification and Fig. 22B depicts Farber mouse bone marrow after administration of rhAC at a dosage of 10 mg/kg/dose at 10x magnification. Surprisingly, the bone marrow of Farber mouse lacked histiocytic cellular infiltrate after administration of rhAC (comparable to a normal mouse), and normal hematopoietic cells in sheets (undisrupted).

The above findings demonstrated, consistent with the studies in Example 1, that ERT in Farber disease mice had a significant impact on several important endpoints, including tissue ceramides, macrophage infiltration, and plasma MCP-1 levels. Further, administration of rhAC unexpectedly prevented marrow histocyte infiltrates, minimized physeal dysplasia, slightly decreased synovial soft tissue infiltrates, and improved proper trabeculae formation. Additional unexpected findings include histocytic infiltration of synovial soft tissue (ligaments and fat pads) and bone marrow, nodular physeal (growth plate) dysplasia (femur), decreased bony trabeculae in epiphysis and metaphysis (femur), and thyroid ablation where samples were available.

Studies will be conducted to delineate the effects of rhAC on macrophage polarization in tissues of Farber mice treated with rhAC. Tissues can be stained with pro-inflammatory markers (e.g., CD38) and anti-inflammatory markers (e.g., CD206) (Jablonski et al., 2015), as well as backup markers (such as, e.g., Erg2 and Fpr2), and pan-macrophage markers (e.g., F4/80) (Dworski et al., 2015). For example, Fig. 23A shows a pan-macrophage stain (F4/80), Fig. 23B shows an anti-inflammatory stain (CD206), and Fig. 23C shows an overlay of Figs. 23A and 23B to show anti-inflammatory macrophages.

References discussed in the application, which are incorporated by reference in their entirety, for their intended purpose, which is clear based upon its context.

Alayoubi, A.M., J.C. Wang, B.C. Au, S. Carpentier, V. Garcia, S. Dworski, S. El-Ghamrasni, K.N. Kirouac, M.J. Exertier, Z.J. Xiong, G.G. Prive, C.M. Simonaro, J. Casas, G. Fabrias, E.H. Schuchman, P.V. Turner, R. Hakem, T. Levade, and J.A. Medin (2013). Systemic ceramide accumulation leads to severe and varied pathological consequences. EMBO Mol Med, 5:827-842.

Bae, J.S., Jang, K.H., Schuchman, E.H., and Jin, H.K. (2004). Comparative effects of recombinant acid sphingomyelinase administration by different routes in Niemann-Pick disease mice. Exp Anim, 53:417-421.

Becker, K.A., Riethmüller, J., Lüth, A., Döring, G., Kleuser, B., and Gulbins, E. (2010). Acid Sphingomyelinase Inhibitors Normalize Pulmonary Ceramide and Inflammation in Cystic Fibrosis. Am. J. Respir. Cell. Mol. Biol., 42(6):716-724.

Bernardo, K., R. Hurwitz, T. Zenk, R.J. Desnick, K. Ferlinz, E.H. Schuchman, and K. Sandhoff (1995). Purification, characterization, and biosynthesis of human acid ceramidase. J Biol Chem, 270:11098-11102.

Boado, R.J., Lu, J.Z., Hui, E.K., Lin, H., and Pardridge, W.M. (2016). Insulin recepotr antibody-alpha-N-acetylglucosaminidase fusin portein penetrates the primate blood-brain barrier and reduces glycosaminoglycans in Sanfillippo type B fibroblasts. Mol. Pharm., 13:1385-92.

Chatelut, M., Harzer, K., Christomanou, H., Feunteun, J., Pieraggi, M.T., Paton, B.C., Kishimoto, Y., O'Brien, J.S., Basile, J.P., Thiers, J.C., Salvayre, R., and Levade, T. (1997). Model SV40-transformed fibroblast lines for metabolic studies of human prosaposin and acid ceramidase deficiencies. Clin Chim Acta, 262:61-76.

Desnick, R.J. and Schuchman, E.H. (2012). Enzyme replacement therapy for lysosomal storage diseases: lessons from 20 years of experience and remaining challenges. Annu Rev Genomics Hum Genet, 13:307-335.

Dworski, S., Berger, A., Furlonger, C., Moreau, J.M., Yoshimitsu, M., Trentadue, J., Au, B.C., Paige, C.J., and Medin, J.A. (2015). Markedly perturbed hematopoiesis in acid ceramidase deficient mice. Haematologica, 100(5):e162-165.

Dworski, S., Lu, P., Khan, A., Maranda, B., Mitchell, J.J., Parini, R., Di Rocco, M., Hugle, B., Yoshimitsu, M., Magnusson, B., Makay, B., Arslan, N., Guelbert, N., Ehlert, K., Jarisch, A., Gardner-Medwin, J., Dagher, R., Terreri, M.T., Lorenco, C.M., Barillas-Arias, L., Tanpaiboon, P., Solyom, A., Norris, J.S., He, X., Schuchman, E.H., Levade, T., and Medin, J.A. (2017). Acid Ceramidase Deficiency is characterized by a unique plasma cytokine and ceramide profile that is altered by therapy. Biochim Biophys Acta, 1863(2):386-394 (Epub Dec. 2016 doi: 10.1016/j.bbadis.2016.11.031).

Eliyahu, E., Park, J.H., Shtraizent, N., He, X., and Schuchman, E.H. (2007). Acid ceramidase is a novel factor required for early embryo survival. FASEB J., 21:1403-9.

Eliyahu, E., N. Shtraizent, K. Martinuzzi, J. Barritt, X. He, H. Wei, S. Chaubal, A.B. Copperman, and E.H. Schuchman (2010). Acid ceramidase improves the quality of oocytes and embryos and the outcome of in vitro fertilization. FASEB J, 24:1229-1238.

Eliyahu, E., N. Shtraizent, R. Shalgi and E.H. Schuchman (2012). Construction of conditional acid ceramidase knockout mice and in vivo effects on oocyte development and fertility. Cellular physiology and biochemistry: international journal of experimental cellular physiology, biochemistry, and pharmacology, 30:735-748.

Farber, S. (1952) A lipid metabolic disorder - disseminated "Lipogranulomatosis" - a syndrome with similarity to, and important difference from, Niemann-Pick and Hand-Schuller-Christian disease. Am. J. Dis. Child, 84:499.

Frohbergh, M.E., Guevara, J.M., Greisamer, R.P., Barbe, M.F., He, X., Simonaro, C.M., and Schuchman, E.H. (2016). Acid ceramidase treatment enhances the outcome of autologous chondrocyte implantation in a rat osteochondral defect model. Osteoarthritis Cartilage, 24:752-762.

Gatt, S. (1963). Enzymic hydrolysis and synthesis of ceramides. J Biol Chem, 238:3131-3133.

He, X., N. Okino, R. Dhami, A. Dagan, S. Gatt, H. Schulze, K. Sandhoff, and E.H. Schuchman (2003). Purification and characterization of recombinant, human acid ceramidase. Catalytic reactions and interactions with acid sphingomyelinase. J Biol Chem, 278(35):32978-32986.

Hollak, C.E. and Wijburg, F.A. (2014). Treatment of lysosomal storage disorders: successes and challenges. J Inherit Metab Dis, 37:587-598.

Jablonski, K.A., Amici, S.A., Webb, L.M., Ruiz-Rosado, Jd.D., Popovich, P.G., Partida-Sanchez, S., and Guerau-de-Arellano, M. (2015). Novel Markers to Delineate Murine M1 and M2 Macrophages. PLoS ONE 10(12):e0145342.

Koch, J., S. Gartner, C.M. Li, L.E. Quintern, K. Bernardo, O. Levran, D. Schnabel, R.J. Desnick, E.H. Schuchman, and K. Sandhoff (1996). Molecular cloning and characterization of a full-length complementary DNA encoding human acid ceramidase. Identification of the first molecular lesion causing Farber disease. J Biol Chem, 271(51):33110-33115.

Li, C.M., J.H. Park, X. He, B. Levy, F. Chen, K. Arai, D.A. Adler, C.M. Disteche, J. Koch, K. Sandhoff, and E.H. Schuchman (1999). The human acid ceramidase gene (asah): Structure, chromosomal location, mutation analysis, and expression. Genomics, 62(2):223-231.

Li, C.M., S.B. Hong, G. Kopal, X. He, T. Linke, W.S. Hou, J. Koch, S. Gatt, K. Sandhoff, and E.H. Schuchman (1998). Cloning and characterization of the full-length cDNA and genomic sequences encoding murine acid ceramidase. Genomics, 50(2):267-274.

Li, C.M., J.H. Park, C.M. Simonaro, X. He, R.E. Gordon, A.H. Friedman, D. Ehleiter, F. Paris, K. Manova, S. Hepbildikler, Z. Fuks, K. Sandhoff, R. Kolesnick, and E.H. Schuchman (2002). Insertional mutagenesis of the mouse acid ceramidase gene leads to early embryonic lethality in homozygotes and progressive lipid storage disease in heterozygotes. Genomics, 79(2):218-224.

Murray JM, Thompson, AM, Vitsky A, Hawes M, Chuang WL, Pacheco J, Wilson S, McPherson JM, Thurberg BL, Karey KP, and Andrews L. (2015). Nonclinical safety assessment of recombinant human acid sphingomyelinase (rhASM) for the treatment of acid sphingomyelinase deficiency: the utility of animal models of disease in the toxicology evaluation of potential therapeutics. Mol Genet Metab, 114:217-225.

Okino, N., He, X., S. Gatt, K. Sandhoff, M. Ito, and E.H. Schuchman (2003). The reverse activity of human acid ceramidase. J Biol Chem, 278(32):29948-29953.

Realini, N., Palese, F., Pizzirani, D., Pontis, S., Basit, A., Bach, A., Ganesan, A., and Piomelli, D. (2015). Acid ceramidase in melanoma: expression, localization and effects of pharmacological inhibition. J Biol Chem, N291:2422-2434.

Roh, J.L., Park, J.Y., Kim, E.H., and Jang, H.J. (2016). Targeting acid ceramidase sensitises head and neck cancer to cisplatin. Eur J Cancer, 52:163-72.

Shiffmann, S., Hartmann, D., Birod, K., Ferreiròs, N., Schreiber, Y., Zivkovic, A., Geisslinger, G., Grösch, S., and Stark, H. (2012). Inhibitors of Specific Ceramide Synthases. Biochimie, 94(2):558-565.

Schuchman, E.H. (2016). Acid ceramidase and the treatment of ceramide diseases. The expanding role of enzyme replacement therapy. Biochim Bipphys Acta, 1862:1459-1471.

Simonaro, C.M., Sachot, S., Ge, Y., He, X., DeAngelis, V.A., Eliyahu, E., Leong, D.J., Sun, H.B., Mason, J.B., Haskins, M.E., Richardson, D.W., and Schuchman, E.H. (2013). Acid ceramidase maintains the chondrogenic phenotype of expanded primary chondrocytes and improves the chondrogenic differentiation of bone marrow-derived mesenchymal stem cells. PLoS One, 8:e62715.

Shtraizent, N., E. Eliyahu, J.H. Park, X. He, R. Shalgi and E.H. Schuchman (2008). Autoproteolytic cleavage and activation of human acid ceramidase. J Biol Chem, 283(17):11253-11259.

Sugita, M., Dulaney, J.T., and Moser, HW (1972). Ceramidase deficiency in Farber's disease (lipogranulomatosis). Science, 178(4065):1100-1102.

Sugita, M., Dulaney, J.T., and Moser, HW (1972). Ceramidase deficiency in Farber's disease (lipogranulomatosis). Science, 178(4065):1100-1102.

The disclosures of each and every patent, patent application, publication, and accession number cited herein are hereby incorporated herein by reference in their entirety.

While present disclosure has been disclosed with reference to various embodiments, it is apparent that other embodiments and variations of these may be devised by others skilled in the art without departing from the true spirit and scope of the disclosure. The appended claims are intended to be construed to include all such embodiments and equivalent variations.

## Claims

1. A pharmaceutical composition comprising a recombinant human acid ceramidase for use in a method of treating Farber disease in a subject in need thereof, the method comprising administering to the subject the pharmaceutical composition comprising a recombinant human acid ceramidase in an effective amount of about 0.1 mg/kg to about 50 mg/kg.

2. The pharmaceutical composition for use according to claim 1, wherein the method of treating Farber disease comprises reducing lipogranulomas, reducing spleen weight, reducing ceramide or increasing sphingosine in a subject with, or suspected of having, Farber disease.

3. A pharmaceutical composition comprising an isolated expressed recombinant human acid ceramidase (rhAC) for use in a method of treating Farber disease in a subject in need thereof, the method comprising:
a. expressing rhAC in a cell;
b. isolating the expressed rhAC from the cell; and
c. administering to the subject a pharmaceutical composition comprising the isolated expressed rhAC in an effective amount of about 0.1 mg/kg to about 50 mg/kg.

4. The pharmaceutical composition for use according to claim 3, wherein the method of treating Farber disease comprises reducing lipogranulomas, reducing spleen weight, reducing ceramide or increasing sphingosine in a subject with, or suspected of having, Farber disease.

5. The pharmaceutical composition for use according to any one of claims 3 or 4, wherein the expressing rhAC in a cell comprises transferring a vector encoding rhAC into the cell.

6. The pharmaceutical composition for use according to claim 5, wherein the vector is a viral vector or a plasmid.

7. The pharmaceutical composition for use according to claim 5, wherein the vector comprises a promoter operably linked to the rhAC.

8. The pharmaceutical composition for use according to claim 5, wherein the vector is transfected into the cell or infected into the cell.

9. The pharmaceutical composition for use according to claim 5, wherein the cell is a Chinese hamster ovarian (CHO) cell or a NS0.

10. The pharmaceutical composition for use according to any one of claims 1-9, wherein the effective amount is about 0.1 mg/kg to about 10 mg/kg, about 10 mg/kg to about 50 mg/kg, about 20 mg/kg to about 30 mg/kg, about 30 mg/kg to about 40 mg/kg or about 40 mg/kg to about 50 mg/kg.

11. The pharmaceutical composition for use according to any one of claims 1-9, wherein the effective amount is about 1, 2, 3, 4, 5, 6, 7, 8, 9, or 10 mg/kg.

12. The pharmaceutical composition for use according to any one of claims 1-11, wherein the effective amount is administered once a week, once every two weeks, once every three weeks, once every four weeks, or once every month to the subject.

13. The pharmaceutical composition for use according to any one of claims 1-12, wherein the pharmaceutical composition is a solution, optionally wherein the pharmaceutical composition comprises cell conditioned media comprising the rhAC.

14. The pharmaceutical composition for use according to any one of claims 1-13, wherein the administration comprises contacting the pharmaceutical composition with the skin of the subject or parenterally administering the pharmaceutical composition to the subject.

15. The pharmaceutical composition for use according to claim 14, wherein parenterally administering the pharmaceutical composition to the subject comprises injecting the pharmaceutical composition to the subject or is an intraperitoneal injection or intravenous injection, or is by intrathecal, intracelebellar, intracerebroventricular, or intraspinal administration.
